# EUROPEAN PATENT SPECIFICATION

(11) **EP 1 715 343 B1**
(45) Date of publication and mention of the grant of the patent: **09.12.2009**
(21) Application number: 06112423.6
(22) Date of filing: 10.04.2006
(51) Int. Cl.: G01N 33/566

(54) **Method for the identification of epitopes related to immunogenicity in biopharmaceuticals**
Verfahren zur Identifizierung von Epitopen in Zusammenhang mit Immunogenität bei Biopharmazeutika
Méthode d'identification des épitopes en rapport avec l'immunogénicité des produits bio-pharmaceutiques

(30) Priority: 20.04.2005 EP 05103199
(43) Date of publication of application: 25.10.2006
(73) Proprietor: F. Hoffmann-La Roche AG, 4070 Basel (CH)
(72) Inventor: Kropshofer, Harald, 79539 Loerrach (DE); Vogt, Anne, 79539 Loerrach (DE)

(56) References cited:
- EP-A- 1 405 862
- WO-A-00/52045
- WO-A-02/22803
- WO-A-94/04557
- WO-A-94/13320
- WO-A-98/33888
- WO-A-2005/014622
- WANG R-F ET AL: "HUMAN TUMOR ANTIGENS FOR CANCER VACCINE DEVELOPMENT" IMMUNOLOGICAL REVIEWS, MUNKSGAARD, vol. 170, 1999, pages 85-100, XP001015479 ISSN: 0105-2896
- DONGRE A R ET AL: "In vivo MHC class II presentation of cytosolic proteins revealed by rapid automated tandem mass spectrometry and functional analyses" EUROPEAN JOURNAL OF IMMUNOLOGY, WEINHEIM, DE, vol. 31, no. 5, May 2001 (2001-05), pages 1485-1494, XP002304910 ISSN: 0014-2980
- TSARK E C ET AL: "Differential MHC class II-mediated presentation of rheumatoid arthritis autoantigens by human dendritic cells and macrophages" JOURNAL OF IMMUNOLOGY, THE WILLIAMS AND WILKINS CO. BALTIMORE, US, vol. 169, no. 11, 1 December 2002 (2002-12-01), pages 6625-6633, XP002304908 ISSN: 0022-1767
- SANTAMBROGIO L ET AL: "Extracellular antigen processing and presentation by immature dendritic cells" PROCEEDINGS OF THE NATIONAL ACADEMY OF SCIENCES OF USA, NATIONAL ACADEMY OF SCIENCE, WASHINGTON, DC, US, vol. 96, no. 26, 21 December 1999 (1999-12-21), pages 15056-15061, XP002222004 ISSN: 0027-8424
- CHICZ R M ET AL: "SPECIFICITY AND PROMISCUITY AMONG NATURALLY PROCESSED PEPTIDES BOUND TO HLA-DR ALLELES" JOURNAL OF EXPERIMENTAL MEDICINE, TOKYO, JP, vol. 178, no. 1, July 1993 (1993-07), pages 27-47, XP002069888 ISSN: 0022-1007

## Description

One aspect that contributes to the immunotoxicity of biological therapeutics is their immunogenicity. Biopharmaceuticals that are immunogenic give rise to antibodies that may lead to potency loss and adverse events, such as allergy, infusion reactions or autoimmunity, in clinical trials. The potential to be immunogenic relies on the presence of T cell epitopes within the sequence of a protein pharmaceutical. This invention relates to a an *in vitro* method for identifying epitopes that may play a causal role in inducing immunogenicity of biopharmaceuticals, such as antibodies or other therapeutic proteins. More specifically, the method of the invention can be used for determining the sequence of immunogenic peptides presented via peptide receptors of dendritic cells which trigger immune reactions leading to immunogenicity. Knowledge about immunogenic epitopes opens the possibility to de-risk therapeutic polypeptides by site-directed mutagenesis with the aim to generate non-immunogenic biopharmaceuticals.

Methods used so far for determining epitopes that may render pharmaceutical proteins immunogenic rely on *in silico* prediction algorithms, *in vitro* screening of overlapping synthetic peptides in T cell activation assays or animal vaccination models. The method of the present invention is based on isolating immunogenic peptides from human dendritic cells that have been pulsed with the respective pharmaceutical protein, and the determination of the sequence of the potential T cell epitopes of the pharmaceutical protein.

Methods for identifying antigenic peptides are known. EP-A-1405862 discloses methods useful for identifying disease-associated antigenic peptides form cells or body fluids derived from a mammalian organism in an amount sufficient to determine their sequence and identity. The antigenic peptides can be utilized for diagnostic or therapeutic purposes and for controlling the quality of vaccines.

WO 2005/014622 A discloses methods for isolating and identifying peptides derived form serum or synovial fluid of patients with Rheumatoid Arthritis (RA). The MHC class II associated RA antigenic peptides can be used as markers on the diagnosis of RA and in therapy as anti-RA vaccines.

Almost any therapeutic protein displays a certain degree of immunogenicity in clinical trials. The initial trigger for immunogenicity is the activation of CD4+ T lymphocytes upon recognition of peptide fragments of the respective pharmaceutical protein. These peptides are referred to as "T cell epitopes" or, briefly, "epitopes".

Activation of CD4+ T cells is only accomplished when T cell epitopes are presented in the context of molecules encoded by the major histocompatibility complex (MHC). In humans, MHC molecules are termed human leukocyte antigens (HLA). HLA-associated peptides are short, encompassing 9-25 amino acids (Kropshofer, H. &Vogt, A. B., Immunol Today 18 (1997) 77-82).

With regard to their function, two classes of MHC- peptide complexes can be distinguished (Germain, R., Cell 76 (1994) 287- 299): (i) MHC class I- peptide complexes can be expressed by almost all nucleated cells in order to attract CD8+ cytotoxic T cells which lyse infected cells or tumor cells, (ii) MHC class II-peptide complexes are constitutively expressed only on so-called antigen presenting cells (APCs), such as B lymphocytes, macrophages or dendritic cells (DCs). In particular, DCs have the capacity to prime CD4+ T helper cells and thereby initiate immunogenicity (Banchereau, J. &Steinman, R. M., Nature 392 (1998) 245-254).

The present invention provides a method for decreasing the immunogenicity of a therapeutic polypeptide comprising
a) identifying the immunogenic peptides of the therapeutic polypeptide comprising
   i) providing cells expressing MHC II molecules in a total amount of 0.1 to 5 µg molecules,
   ii) contacting the cells from (i) with a source of immunogenic peptides,
   iii) isolating MHC II molecule-immunogenic peptide complexes from the cells,
   iv) eluting the associated peptides from the MHC II molecules,
   v) identifying the immunogenic peptides,
   vi) verifying the identified immunogenic peptides as epitopes,
b) modifying the corresponding epitopes of the polypeptide so that the binding of MHCII molecules is reduced or abolished,
c) thereby creating a modified polypeptide with reduced or no immunogenicity potential.

In a preferred embodiment, the MHC II expressing cells are dendritic cells. More preferably, the dendritic cells are exposed to a potential source of immunogen as immature dendritic cells at the same time as they are induced to mature to dendritic cells.

In a further preferred embodiment, the source of potential immunogen belongs to the group comprising polypeptides including therapeutic polypeptides as cytokines, chemokines, growth factors, antibodies, enzymes, structural proteins, hormones or a fragment thereof.

In yet another preferred embodiment, the complexes of MHCII molecules with immunogenic peptides are isolated from the cells with methods comprising solubilization of the cells with a detergent and sequestration of the complexes of MHCII molecules with immunogenic peptides by immunoprecipitation or immunoaffinity chromatography.

In yet another preferred embodiment, the sequestered complexes of MHCII molecules with immunogenic peptides are washed with water in an ultrafiltration tube before eluting the peptides.

In yet another preferred embodiment, the immunogenic peptides are eluted from the MHCII molecules using diluted acid.

In yet another preferred embodiment, the isolated immunogenic peptides of step (a)(iv) are fractionated and sequenced. More preferably, the isolated immunogenic peptides are fractionated and sequenced by methods comprising liquid chromatography and mass spectrometry.

In yet another preferred embodiment, the isolated immunogenic peptides are identified by comparing the peptide identified from cells which have been contacted with a source of potential immunogen with those, which have been identified from cells which have not been contacted with that source.

The preferred immunogenic peptides are naturally-processed immunogenic peptides.

### Method

Step a) of the above described method concerns a method for isolating and identifying immunogenic peptides. The method of step a) provides complexes of peptide receptors with potentially immunogenic peptides in an amount of 0.1 to 5 µg, preferably in an amount of 0.2 to 3 µg. This quantity equals to the amount of material which is normally available from DCs cells obtained from peripheral blood of patients or healthy donors. The lowest amount of material necessary in the prior art is about 200 µg MHC class II molecules derived from an unlimited source (inbred mice) (Dongre A R et al., EJI 2001, 31, 1485-94). This is about two orders of magnitude more material than available from human peripheral blood.

Specifically, step a) of the above described method of the invention comprises the steps of
i) providing cells expressing MHC II molecules in a number providing 0.1 to 5 ug MHC II molecules, preferably, in a number providing 0.2 to 3 ug,
ii) contacting the cells from (i) with a source of immunogenic peptides,
iii) isolating MHC II molecule-immunogenic peptide complexes from the cells,
iv) eluting the associated peptides from the MHC II molecules,
v) identifying the immunogenic peptides,
vi) validating the identified immunogenic peptides as epitopes.

Preferably, the step a) of the above described method of the invention comprises the steps of
i) providing cells expressing MHC II molecules in a number providing 0.1 to 5 ug MHC II molecules, preferably, in a number providing 0.2 to 3 ug,
ii) contacting the cells from (i) with a source of immunogenic peptides,
iii) sequestering the MHC II molecule-immunogenic peptide complexes from the cells by immunoprecipitation or immunoaffinity chromatography,
iv) washing the bounded complexes of MHC II molecules with antigenic peptides with water or low salt buffer,
v) eluting the associated peptides from the MHC II molecules,
vi) identifying the immunogenic peptides,
vii) validating the identified immunogenic peptides as epitopes.

The method for decreasing the immunogenicity of a polypeptide further comprises
b) modifying the corresponding epitopes of the polypeptide so that the binding of MHCII molecules is reduced or abolished
c) thereby creating mutated polypeptide with reduced or no immunogenicity potential.

The term "polypeptide" as used herein refers to a chain of linked amino acids.

The term "immunogenicity" as used herein refers to the quality of a substance which is able to provoke an immune response against the substance. A measure of how able the substance is at provoking an immune response against it.

The term "immunogenicity potential" as used herein refers to potential capacity of a polypeptide to elicit an immune response.

The term "immune response" as used herein refers to a bodily defense reaction that recognizes an invading substance and produces antibodies specific against that antigen

The amount of tissue or bodily fluid necessary to obtain e.g. 100 ng MHC class II molecules depends on the number of cells that do express MHC class II and on the expression rate of MHC class II molecules: e.g. 100 ng of MHC class II are equivalent to about 2×10⁵ mature DCs or 5 to 10×10⁶ peripheral blood monocytes or about 5×10⁷ peripheral blood mononuclear cells which can be obtained from about 50 ml of blood.

The high sensitivity required for identifying MHC class II associated peptides is explained by the fact that each type of these peptide receptors, e.g. human MHC class II gene product HLA-DR1, carries about 500 to 1000 different antigenic peptides (Chicz R M et al., J Exp. Med. 1993, 178, 27-47; Chicz R M & Urban R G, Immunol. Today, 1993, 15: 155-160). However, most of the 500 to 1000 different peptides attain very low copy numbers and, therefore, are not very likely to play a physiological role. Especially in the MHC class II field, those peptides that are of immunological relevance e.g. those that activate helper T cells and thereby facilitate immunogenicity of pharmaceutical proteins, attain moderate to high copy numbers (Latek R R & Unanue E R, Immunol. Rev. 1999, 172: 209-228). These peptides cover about 40 to 50% of the total amount of peptide material eluted from MHC class II molecules and equal to about 10 to 20 individual peptides.

Many MHC class II associated peptides are represented as a set of 2 to 5 C- and N-terminal truncation variants (Rudensky A Y et al, Nature 1992, 359, 429-431; Chicz et al. Nature 1992, 358: 764-768) sharing a common core sequence of about 10 to 13 amino acids which is essential for recognition by the T cell receptor. These truncation/elongation variants constitute the same T cell epitope. This means that the number of different epitopes, which are of importance is actually smaller, ranging from about 5 to 70 different epitopes. Thus, the abundance of immunogenic epitopes ranges from 0.2% to 5%.

### Origin of the Peptides

The antigenic peptides are peptides which are associated with MHC class II molecules on the surface of human DCs. The antigenic peptides may be bound to intra- or extracellular MHC class II molecules. The term "immunogenic peptide" as used herein refers to an antigenic peptide which may elicit an immune response. The immunogenic peptides may derive from polypeptides after coincubation with DCs. The polypeptides which are a potential source of immunogenic peptides are polypeptides including therapeutic polypeptides such as cytokines (i.e. interferones, interleukins, erythropoietin (Epo), granulocyte/ macrophage colony-stimulating factor (GM-CSF) or tumor necrosis factor (TNF)), chemokines, growth factors, antibodies (i.e. monoclonal, polyclonal, chimeric and humanized antibodies), enzymes, structural elements, hormones and fragments thereof.

As all these peptide receptors are able to accommodate a broad variety of peptide ligands (see above), each single peptide whose sequence has to be determined is represented in only femtomolar amounts. 1 µg MHC class II (16 pmol) may carry dominant peptide species, with each single peptide attaining an occupancy of 0.1-2%, which equals to about 16-320 femtomoles. The methods of the present invention allow the isolation of these femtomolar amounts of potentially immunogenic peptides from 0.1 to 5 µg of MHCII molecules loaded with peptides and their subsequent sequencing.

### Origin of the MHCII molecules

The term "Antigen presenting receptors" or "APR" as used herein refers to a peptide receptor which binds antigenic peptides and presents them to other immunological cells and thereby mediating a specific humoral immune response. The antigen presenting receptors used in the method of the invention are MHC class II molecules. MHC class II molecules include but are not limited to HLA-DR, HLA-DQ and HLA-DP molecules. Alternative APR that may play a role are the receptors of the CD 1 family or other so far undefined receptors that present potentially immunogenic peptides to CD4+ helper T cells.

### Origin of the Cellular Material

Cells that express Antigen presenting receptors and at the same time are able to prime or activate CD4+ T cells are referred to as antigen presenting cells (APCs) (Unanue, E. R.. Macrophages, antigen presenting cells and the phenomena of antigen handling and presentation. In: Fundamental Immunology, 2nd edition (editor Paul, W. E) New York, Raven Press, 1989). APCs to be used comprise human B cells, human macrophages, preferentially human dendritic cells. Apart from that, cell mixtures that contain APC, such as peripheral blood mononuclear cells (PBMC) or peripheral blood lymphocytes (PBL) may be used.

The APCs used in the method of the present invention are cells expressing MHC class II molecules. Preferred cells expressing MHC II molecules are dendritic cells.

In order to judge the immunogenicity of polypeptides with respect to a certain population, a series of HLA-typed dendritic cells are to be used, with the HLA types representing the HLA frequencies of the whole population. For example, to cover the Caucasian population with regard to the HLA polymorphism at the HLA-DR locus, dendritic cells derived from about 15-20 blood donors differing in their HLA-DR genotype have to be analysed for potentially immunogenic peptides.

### Solubilization of MHC II molecules from Cells

For the purification of MHC II molecules-peptide complexes from cells, the membranes of the cells have to be solubilized. Cell lysis may be carried out with methods known in the art, e.g. freeze-and- thaw cycles and the use of detergents, and combinations thereof. Preferred lysis methods are solubilization using detergents, preferably TX-100, NP40, n-octylglucoside, Zwittergent, Lubrol, CHAPS, most preferably TX-100 or Zwittergent 3-12. Cell debris and nuclei have to be removed from cell lysates containing the solubilized MHC II molecule-peptide complexes by centrifugation. Therefore, in a further embodiment of the present invention, the complexes of MHCII molecules with immunogenic peptides are isolated from the cells with methods comprising solubilization with a detergent.

### Nano-Scale Purification of MHC-Peptide Complexes

The MHC II-peptide complexes can be purified from cell lysates by methods comprising immunoprecipitation or immunoaffinity chromatography. For the immunoprecipitation or immunoaffinity chromatography, antibodies specific for MHC class II molecules and suitable for these methods are used. The specific antibodies are preferably monoclonal antibodies, and are covalently or non-covalently e.g. via Protein A, coupled to beads, e.g. sepharose or agarose beads. A selection of the broad panel of anti-HLA antibodies used in the prior art comprises:
anti-HLA-DR antibodies: L243, TU36, DA6.147, preferably L243; anti-HLA-DQ antibodies: SPVL3, TU22, TU169, preferably TU22 and TU169; anti-HLA-DP antibody B7/21.
Monoclonal antibodies specific for different MHC class II molecules may be commercially obtained (e.g. Pharmingen, Dianova) or purified from the supernatant of the respective hybridoma cells using Protein A- or Protein G-affinity chromatography. Purified monoclonal antibodies may be coupled by various methods known in the art, preferably by covalently coupling antibody amino groups to CNBr-activated sepharose.

Immunoisolation of MHC molecules may be performed by incubating the antibody-beads with the cell lysate under rotation for several hours or chromatographically by pumping the cell lysate through a micro-column. Washing of the antibody-beads may be performed in eppendorf tubes or in the microcolumn. The efficacy of the immunoprecipitation may be analysed by SDS-PAGE and western blotting using antibodies recognizing denatured MHC molecules (anti-HLA-DRalpha: 1B5).

### Elution and Fractionation of MHC II molecules-Associated Peptides

By eluting the peptides from the receptor molecules, a complex mixture of naturally processed peptides derived from the source of potential immunogen and from polypeptides of intra- or extracellular origin, is obtained. Only after elution, peptides can be fractionated and subjected to sequence analysis.

The term "immunogen" as used herein refers to any polypeptide that provokes an immune response when introduced into the body

The immunogenic peptides in the methods of the present invention may be eluted by a variety of methods known in the art, preferably by using diluted acid, e.g., diluted acetonitrile (Jardetzky T S et al., Nature 1991 353, 326-329), diluted acetic acid and heating (Rudensky A Y et al., Nature 1991, 353, 622-626; Chicz R M et al., Nature 1992, 358, 764-768) or diluted trifluoro acetic acid at 37° C. (Kropshofer H et al., J Exp Med 1992, 175, 1799-1803). Most preferably, the peptides are eluted at 37° C. with diluted trifluoro acetic acid.

In a further embodiment, the sequestered MHCII molecules-peptide complexes are washed with water or low salt buffer before elution in order to remove residual detergent contaminants. The low salt buffer may be a Tris, phosphate or acetate buffer in a concentration range of 0.5-10 mM, preferably in a concentration of 0.5 mM. In a more preferred embodiment, the MHC II molecule-peptide complexes are washed with ultrapure water (sequencing grade) conventionally used for HPLC analysis, preferably with ultrapure (sequencing grade) water from MERCK. The washing step may be carried out by ultrafiltration. The ultrafiltration may be carried out in an ultrafiltration tube with a cut- off of 30 kD, 20 kD, 10 kD or 5 kD, preferably of 30 kD and a tube volume of 0.5-1.0 ml ("Ultrafree" tubes; Millipore). The washing in the ultrafiltration tube may be carried out 4 to 12 times, preferably 6 to 10 times, with a volume of 10 to 20 times the volume of the beads carrying the receptor-peptide complexes, preferably with a volume of 15 times the beads. The eluted peptides may be separated from the remaining MHC II molecules using the same ultrafiltration tube. The eluted peptides may then be lyophilized. Peptide sequence analysis by liquid chromatography-mass spectrometry (LC-MS)

In a further embodiment of the present invention, the isolated immunogenic peptides are fractionated, sequenced and identified. By sequencing it is understood that the amino acid sequence of the individual peptides in the mixture of isolated immunogenic peptides is elucidated by methods adequate to sequence femtomolar amounts of peptides. By identifying it is understood that it is established from which proteins or polypeptides the immunogenic peptides are derived and which sequence they constitute within these proteins or polypeptides.

In a first step, the complex mixture of eluted peptides may be fractionated by one of a variety of possible chromatographic methods, e.g. by reversed phase, anion exchange, cation exchange chromatography or a combination thereof. Preferably, the separation is performed by C18- reverse phase chromatography or by reversed- phase/cation exchange two- dimensional HPLC, denoted as MudPit (Washburn M P et al., Nat Biotechnol., (2001), 19, 242-247).

The fractionation may be done in a HPLC mode utilizing fused- silica micro-capillary columns which are either connected to a nano-flow electrospray source of a mass spectrometer or to a micro-fractionation device which spots the fractions onto a plate for MALDI analysis.

A variety of mass spectrometric techniques are suitable, preferably MALDI- post source decay (PSD) MS or electrospray ionization tandem mass spectrometry (ESI-MS), most preferably ion-trap ESI-MS.

The sequences of the individual peptides can be determined by means known in the art. Preferably, sequence analysis is performed by fragmentation of the peptides and computer-assisted interpretation of the fragment spectra using algorithms, e.g. MASCOT or SEQUEST. Both computer algorithms use protein and nucleotide sequence databases to perform cross- correlation analyses of experimental and theoretically generated tandem mass spectra. This allows automated high through-put sequence analysis.

### Qualitative Peptide Analysis by MALDI Mass Spectrometry

For qualitative analysis of the whole peptide repertoire obtained upon elution, matrix-assisted laser desorption and ionization time-of-flight (MALDI-TOF) mass spectrometry may be carried out. Using settings that do not fragment the peptides, MALDI-TOF analysis provides a rough overview with regard to the complexity of the peptide mixture and the presence of dominant peptides.

### Quantitative Peptide Analysis

To estimate the quantity of single peptides eluted from MHC II molecules, the run through of the micro-capillary column may be analyzed by a flow- through UV detector operated at a detection wave- length of 214 nm. For quantitation the peak areas of peptides to be analyzed are compared with peak areas of graded amounts of synthetic standard peptides.

### Strategy

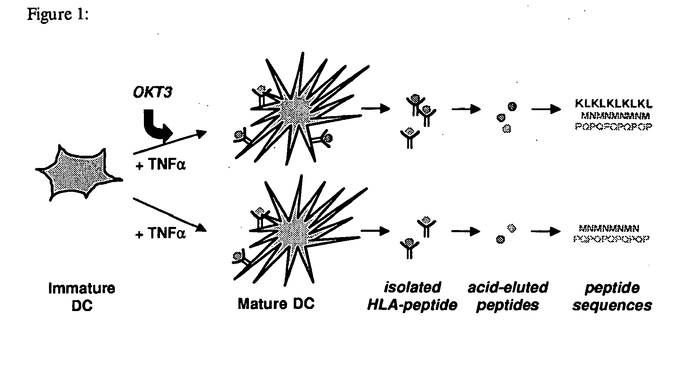

The strategy of the present invention foresees identification of immunogenic peptides which have been loaded onto MHCII molecules of APCs in cell culture (in vitro approach, FIG. 1).

Step a) of the method of the invention is a method for identifying peptides involved in immunogenicity comprising the steps of
i) providing cells expressing MHCII molecules in a number providing 0.1 to 5 ug receptors, preferably, in a number providing 0.2 to 3 ug,
ii) contacting the cells from (i) with a source of immunogenic peptides,
iii) isolating MHCII-immunogenic peptide complexes from the cells,
iv) eluting the associated peptides from the MHCII molecules
v) identifying the immunogenic peptides
vi) validating the identified immunogenic peptides as epitopes.

Preferably, the cells expressing MHC II molecules are dendritic cells, more preferably, the cells expressing MHC II molecules are immature dendritic cells, most preferably, the cells expressing MHC II molecules are immature dendritic cells generated from peripheral blood monocytes.

Dendritic cells may be generated from peripheral blood monocytes or from bone marrow-derived CD34+ stem cell-precursors. The peripheral blood mononuclear cells (PBMCs) may be isolated from blood samples by density gradient centrifugation. The monocytes may then be isolated from PBMCs by methods known in the art, e.g. by sorting with magnetic beads. The source of dendritic cells may be mammalian species, preferably humans. The monocytes may then be differentiated in cell culture to become immature dendritic cells. The differentiation state may be monitored by flow-cytometric analysis, e.g. using upregulation cell surface markers CD83, CD80, CD86, HLA-DR.

The amount of cells necessary to obtain e.g. 100 ng MHC class II molecules depends on the number of cells that do express MHC class II and on the expression rate of MHC class II molecules: e.g. 100 ng of MHC class II are equivalent to about 2×10⁵ mature DCs or 5 to 10×10⁶ peripheral blood monocytes or about 5×10⁷ peripheral blood mononuclear cells which can be obtained from about 50 ml of blood. The cells expressing MHC II molecules are then contacted with a source of therapeutic protein. The cells expressing MHC II molecules, preferably the immature dendritic cells, are at the same time triggered to mature by methods known in the art, e.g. incubation with inflammatory cytokines, like TNF alpha or a mixture ofTNF alpha, IL-6, IL-1 beta, PGE2.

The source of therapeutic protein offered to the cells expressing MHC II molecules may be selected from the group comprising unformulated or formulated protein. Control cells expressing MHC II molecules are treated equivalently except that they are not exposed to the therapeutic protein (cf. Figure 1).

The cells expressing MHC II molecules may be contacted with the polypeptide or a fragment thereof which is taken up by the cells expressing MHC II molecules by receptor- mediated uptake or by fluid phase uptake and internalized.

By eluting the peptides from the MHC II molecules, a set of naturally processed peptides derived from the polypeptide or a fragment thereof is obtained. This polypeptide may be the therapeutic polypeptide of choice or an irrelevant polypeptide of intracellular (a self protein expressed in the cell expressing MHC II molecules in the absence of pulsed therapeutic polypeptide) or extracellular origin (a protein derived from the cell culture medium also present in the absence of pulsed therapeutic polypeptide).

The isolated immunogenic peptides may be identified by comparing the peptide identified from cells which have been contacted with a source of potential immunogen with those, which have been identified from cells which have not been contacted with that source (control).

### Epitope Validation for MHC-Associated Peptides

The peptide sequences identified in step a) of the method of the invention may be validated by one of several criteria, comprising MHC binding motif, MHC binding capacity and recognition by CD4+ T lymphocytes.

MHC binding motifs are common structural characteristics of peptides associated to a particular MHC molecule (allelic variant) which are necessary to form stable complexes with MHC molecules. In the case of MHC class II molecules, the peptide length varies from 12 to 18 amino acids and even longer peptides can bind since both ends of the peptide binding groove are open. Most HLA class II molecules accommodate up to 4 residues relevant for binding, denoted as "anchor residues", at relative positions P1, P4, P6 and P9 contained in a nonameric core region. This core region, however, can have variable distance from the N-terminus of the peptide. In the majority of cases, 2- 4 N-terminal residues precede the core region. Hence, the P1 anchor residues is located at positions 3, 4 or 5 in most HLA class II associated peptides. Peptides eluted from HLA-DR class II molecules share a big hydrophobic P1 anchor, represented by tyrosine, phenylalanine, tryptophane, methionine, leucine, isoleucine or valine.

The position and the exact type of anchor residues constitute the peptide binding motif which is known for most of the frequently occurring HLA-DR class II allelic products. A computer algorithm allowing motif validation in peptide sequences is "Tepitope", available by www.vaccinome. com (by J. Hammer, Nutley, USA).

The MHC II binding capacity of the peptides identified in step a) of the methods of the present invention may be tested by methods known in the art using, for example, isolated MHC class II molecules and synthetic peptides with amino acid sequences identical to those identified in step a) of the method of the invention (Kropshofer H et al., J. Exp. Med. 1992; 175, 1799-1803; Vogt A B et al., J. Immunol. 1994; 153, 1665-1673; Sloan V S et al., Nature 1995; 375, 802-806). Alternatively, a cellular binding assay using MHC class II expressing cell lines and biotinylated peptides can be used to verify the identified epitope (Arndt S O et al., EMBO J., 2000; 19, 1241-1251)

In both assays, the relative binding capacity of a peptide is measured by determining the concentration necessary to reduce binding of a labeled reporter peptide by 50% (IC50). Peptide binding with a reasonable affinity to the relevant HLA class II molecules attains IC50 values not exceeding 10-fold the IC50 of established reference peptides.

The same binding assays can also be used to test the ability of peptides to bind to alternative class II MHC molecules, i.e., class II MHC molecules other than those from which they were eluted using the method of the invention.

The capacity to prime CD4+ T cells represents the most critical epitope verification procedure. This procedure involves testing of peptides identified in step a) of the methods of the invention for their ability to activate CD4+ T cell populations. Peptides with amino acid sequences either identical to those identified in step a) of the methods of the invention or corresponding to a core sequence derived from a nested group of peptides identified in step a) of the methods of the invention are synthesized. The synthetic peptides are then tested for their ability to activate CD4+ in the context auf autologous dendritic cells, expressing the MHC class II molecule of interest.

CD4+ T cell responses can be measured by a variety of in vitro methods known in the art. For example, whole peripheral blood mononuclear cells (PBMC) can be cultured with and without a candidate synthetic peptide and their proliferative responses measured by, e.g., incorporation of [3H]-thymidine into their DNA. That the proliferating T cells are CD4+ T cells can be tested by either eliminating CD4+ T cells from the PBMC prior to assay or by adding inhibitory antibodies that bind to the CD4+ molecule on the T cells, thereby inhibiting proliferation of the latter. In both cases, the proliferative response will be inhibited only if CD4+ T cells are the proliferating cells. Alternatively, CD4+ T cells can be purified from PBMC and tested for proliferative responses to the peptides in the presence of APC expressing the appropriate MHC class II molecule. Such APCs can be B-lymphocytes, monocytes, macrophages, or dendritic cells, or whole PBMC. APCs expressing MHC II molecules can also be immortalized cell lines derived from B-lymphocytes, monocytes, macrophages, or dendritic cells. The APCs can endogenously express the MHC class II molecule of interest or they can express transfected polynucleotides encoding such molecules. In all cases the APCs expressing MHCII molecules can, prior to the assay, be rendered non-proliferative by treatment with, e.g., ionizing radiation or mitomycin-C.

As an alternative to measuring cell proliferation, cytokine production by the CD4+ T cells can be measured by procedures known to those in art. Cytokines include, without limitation, interleukin- 2 (IL-2), interferon- gamma (IFN-gamma), interleukin-4 (IL-4), TNF-alpha, interleukin-6 (IL-6), interleukin-10 (IL-10), interleukin-12 (IL-12) or TGF-beta. Assays to measure them include, without limitation, ELISA, ELISPOT and bioassays in which cells responsive to the relevant cytokine are tested for responsiveness (e.g., proliferation) in the presence of a test sample.

### Applications

The methods of the present invention can be applied to identify peptides involved in the immunogenicity of any biopharmaceutical drug, especially those in which unacceptable potency loss is due to neutralizing anti-drug antibodies or where adverse or severe adverse events in clinical trials are thought to rely on immunogenicity and to de-risk the respective (therapeutic) polypeptides with regard to their immunogenicity. De-risking may be accomplished by exchange of one or more anchor residues critical for binding to MHC class II molecules, thereby creating mutated therapeutic polypeptides that have reduced or no immunogenicity potential. Alternatively, residues critical for recognition by the T cell receptor on CD4+ T cells can be exchanged.

Methods for exchanging anchor residues critical for binding to MHC class II molecules are well known in the art, i.e. replacement of the P1 anchor of a HLA-DR1-restricted T cell epitope by alanine, glycine, proline or a charged residue (cf. Kropshofer et al., EMBO J. 15, 6144-6154; 1996).

The methods of this invention can be used to reduce the number of epitopes that are being identified through *in silico* epitope prediction algorithms. Prediction codes tend to over-predict the number of epitopes contained in therapeutic polypeptides. The consequence of such an over-prediction is that de-risking of high numbers of predicted epitopes may lead to loss of bioactivity in those cases where certain sequence stretches confer both bioactivity and immunogenicity. As the present invention identifies naturally presented peptide epitopes, which have undergone competition for MHC binding sites and quality control by the peptide editor HLA-DM inside the APC, the methods presented here narrow down the number of potential epitopes to a reasonably small number. De-risking of a reduced number of epitopes will more likely retain the bioactivity of therapeutic polypeptides.

Having now generally described this invention, the same will become better understood by reference to the specific examples, which are included herein for purpose of illustration only and are not intended to be limiting unless otherwise specified, in connection with the following figures.

### Figures

Figure 1 shows a diagram of the methodology to study naturally processed MHC class II-associated peptide epitopes derived from a therapeutic polypeptide added to human dendritic cells.

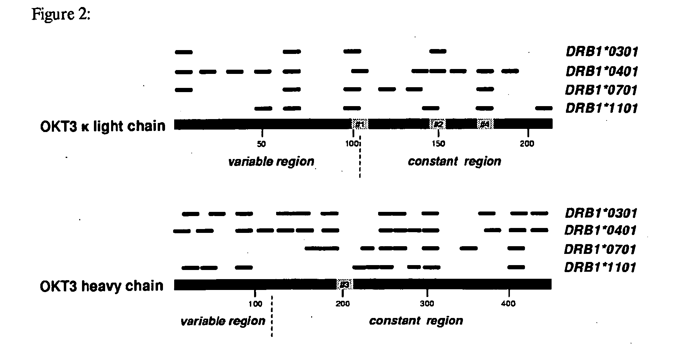

Figure 2 show a comparison of OKT3-derived peptide epitopes identified through the in silico prediction algorithm TEPITOPE versus the in vitro methodology involving dendritic cells. Potential T cell epitopes were predicted for the HLA-DRB1 alleles *0301, *0401, *0701 and *1101, as indicated by small black rectangles above the protein sequence. The threshold for the TEPITOPE analysis was set to 1-4%. The signal peptide of unprocessed OKT3 light chain was omitted. The epitopes identified by the cellular in vitro technology are marked by numbers and boxes in the OKT3 sequence.

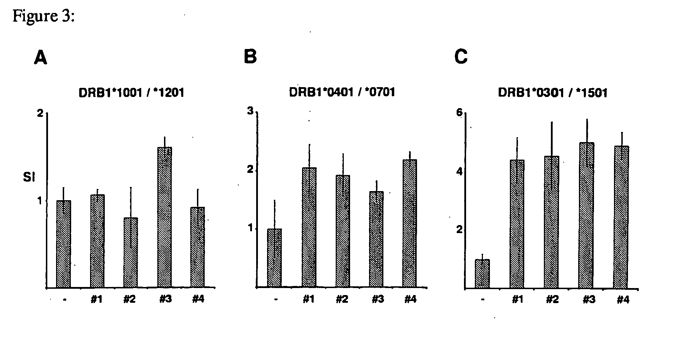

Figure 3 shows a diagram of CD4+ T cell activation by synthetic OKT3-derived peptides #1-4. The intensity of T cell activation is indicated by the stimulation index (SI). The sequences of the peptides used for stimulation (10 uM each) were as follows: #1, OKT3-1c 98-113, GSGTKLEINRADTAPT, #2, OKT-1c 143-158, INVKWKIDGSERQNGV, #3, OKT3-hc 194-209, WPSQSITCNVAHPASS, #4, OKT3-1c 164-183, DQDSKDSTYSMSSTLTLTKDE. T cells were re-stimulated once (B) or twice (A,C) with the respective peptide and mature dendritic cells. The HLA-DRB1 genotypes of the dendritic cells and T cells employed are indicated on top of each diagram. Error bars indicate SD obtained with 3 independent experiments. The average SI in the absence of added peptide was adjusted to 1.0. Donor cells with the following DRB1 haplotype were used: *0401 / *0701 (A), *0301 / *1501 (B), and *1001 / *1201 (C)

### EXAMPLES

The examples below are in connection with the figures described above and based on the methodology summarized in FIG. 1 and described in detail in the following. Commercially available reagents referred to in the examples were used according to manufacturer's instructions unless otherwise indicated.

### Methodology of the Invention

### Cell Lines and Culture

The study was performed with human dendritic cells which were differentiated from monocytes, as described below. Monocytes were purified from human peripheral blood. All cells were cultured in RPMI 1640 medium (short: RPMI) supplemented with 1 mM Pyruvat, 2 mM Glutamine and 10% heat-inactivated fetal calf serum (Gibco BRL, Rockville, Md.).

### Isolation of Peripheral Blood Mononuclear Cells (PBMCs)

Peripheral blood was obtained from the local blood bank as standard buffy coat preparations from healthy donors. Heparin (200 I.U./ml blood, Liquemine, Roche) was used to prevent clotting. Peripheral blood mononuclear cells (PBMCs) were isolated by centrifugation in LSM®(1.077- 1.080 g/ml; ICN, Aurora, Ohio) at 800 g (room temperature) for 30 min. PBMCs were collected from the interphase and washed twice in RPMI containing 20 mM Hepes (500 g for 15 min, 300 g for 5 min). In order to remove erythrocytes, PBMCs were treated with ALT buffer (140 mM ammonium chloride, 20 mM Tris, pH 7.2) for 3 min at 37° C. PBMCs were washed twice with RPMI containing 20 mM Hepes (200 g for 5 min).

### HLA-typing of Peripheral Blood Monocytes

The HLA-DR genotype of PBMCs used for isolation of monocytes and differentiation of dendritic cells was determined by Roche Molecular Systems (Alameda, CA, USA).

### Generation of Dendritic Cells from Peripheral Blood Monocytes

Monocytes were isolated from PBMCs by positive sorting using anti-CD 14 magnetic beads (Miltenyi Biotech, Auburn, Calif.) according to the manufacturer's protocol. Monocytes were cultured in RPMI supplemented with 1% non-essential amino acids (Gibco, BRL, Rockville, Md.), 50 ng/ml recombinant human granulocyte macrophage-colony stimulating factor (GM- CSF; S.A. 1.1×10 ⁷U/mg) (Leucomax; Novartis, Basel Switzerland) and 3 ng/ml recombinant human IL-4 (S.A. 2.9×10⁴U/mg) (R&D Systems, Minneapolis, Minn.). Monocytes were seeded at 0. 3×10⁶/ml in 6- well plates (Costar) for 5 days to obtain immature dendritic cells.

The quality of monocyte-derived immature dendritic cells was routinely monitored by flow-cytometric analysis conforming to the phenotype: CD1a (high), CD3 (neg.), CD14 (low), CD19 (neg.), CD56 (neg.), CD80 (low), CD83 (neg.), CD86 (low) and HLA DR (high). In contrast, mature dendritic cells (cf. below) display the following phenotype: CD1a (low), CD80 (high), CD83 (high), CD86 (high) and HLA-DR (high). Monoclonal antibodies against CD1a, CD3, CD14, CD19, CD56, CD80, CD83, CD86 as well as the respective isotype controls were purchased from Pharmingen (San Diego, Calif.).

### Exposure of Dendritic Cells to the therapeutical polypeptide

To facilitate the uptake of the pharmaceutical protein by dendritic cells, 6× 10⁶ immature dendritic cells were exposed to 5-50 ug of the biopharmaceutical. At the same time, maturation of dendritic cells was induced by adding 10 ng/ml recombinant human tumor necrosis factor (TNFalpha; S.A. 1.1×10⁵ U/mg). As a control, 6×10⁶ dendritic cells were incubated with TNFalpha alone (Figure 1)

After 24-48 hrs of co-culture, mature dendritic cells were harvested by centrifugation at 300 g for 10 min. Cells were washed with RPMI containing 10% FCS and transferred to an eppendorf tube. After centrifugation at 400 g for 3 min, the supernatant was completely removed and the cells were frozen at -70°C.

### Generation of Anti-HLA Class II Beads

The anti-HLA-DR monoclonal antibody (mAb) L243 (ATCC, Manassas, Va.) was produced by culturing the respective mouse hybridoma cell line. mAb L243 was purified using ProteinA sepharose (Pharmacia, Uppsala, Sweden) and immobilized to CNBr-activated sepharose beads (Pharmacia) at a final concentration of 2.5 mg/ml, according to the manufacturer's protocol. L243 beads were stored in PBS containing 0.1% Zwittergent 3-12 (Calbiochem, La Jolla, Calif.).

### Nano-Scale Purification of HLA-DR-Peptide Complexes

Pellets of frozen dendritic cells were resuspended in 10- fold volume of ice cold lysis buffer (1% Triton-X-100, 20 mM Tris, pH 7.8, 5 mM MgCl₂, containing protease inhibitors chyrnostatin, pepstatin, PMSF and leupeptin (Roche, Mannheim, Germany)) and lysed in a horizontal shaker at 1000 rpm, 4° C. for 1 h. The cell lysate was cleared from cell debris and nuclei by centrifugation at 2000 g, 4° C. for 10 min. The lysate was co-incubated with L243 beads (5-10 µl L243 beads per 100 µl cell lysate) in a horizontal shaker at 1000 rpm, 4° C. for 2 hrs. Immunoprecipitated HLA-DR-peptide complexes bound to L243 beads were sedimented by centrifugation at 2000 g, 4° C. for 5 min and washed three times with 300 µl 0.1% Zwittergent 3-12 (Calbiochem) in PBS.

The efficacy of depletion of HLA-DR-peptide complexes was monitored by analyzing the respective cell lysates before and after immunoprecipitation. In parallel, aliquots of the beads were analyzed by western blotting using the anti-HLA-DRa-specific mAb 1B5 (Adams, T. E. et al., Immunology 50 (1983) 613-624).

### Elution of HLA-DR-Associated Peptides

HLA-DR-peptide complexes bound to L243 beads were resuspended in 400 µl H ₂O (HPLC-grade; Merck, Darmstadt, Germany), transferred to an ultrafiltration tube, Ultrafree MC, 30 kD cut- off (Millipore, Bedford, Mass.) and washed 10 times with 400 µl H₂O (HPLC-grade) by centrifugation for 2-4 min at 14000 rpm at 4° C. For eluting the bound peptides, 50 µl 0.1% trifluoracetic acid (Fluka, Buchs, Switzerland) in H₂O (HPLC-grade) was added and incubation was performed for 30 min at 37° C. Fluted peptides were collected in a new eppendorf tube by centrifugation of the ultrafiltration tube at 14000 rpm for 3 min at RT and immediately lyophilized in a Speed-Vac®vacuum centrifuge.

### Fractionation of Peptides by Nano-HPLC

Lyophilized peptides eluted from HLA-DR molecules were resolved in 0.05% trifluoroacetic acid, 5% acetonitrile (Merck, Darmstadt, Germany) in H₂O, (HPLC-grade) and separated on a 75 µm×15 cm C18 PepMap capillary (C18; 3 µm; 100 Å) (LC-Packings, Amsterdam, Netherlands) connected to a FAMOS®autosampler and an ULTIMATE®nano-flow HPLC (Dionex, Olten, Switzerland). The following non- linear gradient at a constant flow rate of 200 nl/min was used: 0-40 min 5-50% system B; 40-50 min 50-90% system B. System A was 0.05% trifluoroacetic, 5% acetonitrile/H₂O and system B was 0.04% trifluoroacetic, 80% acetonitrile/H₂O. The separation was monitored via dual UV absorption at 214 nm and 280 nm. Fractions (400 nl) were collected using the fraction collector PROBOTT (BAI, Weiterstadt, Germany) and spotted onto an AnchorChip 600/384 MALDI-MS target (Bruker, Bremen, Germany).

### Sequence Analysis of Peptides by Ion Trap MS/MS Mass Spectrometry

To perform high-throughput sequencing of complex peptide mixtures, the MudPIT (multidimensional protein identification technology) was used (Washburn M P et al., Nat Biotechnol 19 (2001), 242-247) which is based on a liquid chromatographic fractionation followed by mass spectrometric sequencing.

To this end, the lyophilized peptides eluted from HLA molecules were resuspended in a buffer containing 5% (v/v) acetonitrile, 0.5% (v/v) acetic acid, 0.012% (v/v) heptafluoro butyric acid (HFBA) and 5% (v/v) formic acid. The sample was separated on a fused-silica microcapillary column (100 µm i.d.×365 µm) generated by a Model P- 2000 laser puller (Sutter Instrument Co., Novato, Calif.). The microcolumn was packed with 3 µm/C18 reverse-phase material (C18-ACE 3 µm [ProntoSIL 120-3-C18 ACE-EPS, Leonberg, Germany]) followed by 3 cm of 5 µm cation exchange material (Partisphere SCX;Whatman, Clifton, N.J.).

A fully automated 8-step gradient separation on an Agilent 1100 series HPLC (Agilent Technologies, Waldbronn, Germany) was carried out, using the following buffers: 5% ACN/0.02% HFBA/0.5% acetic acid (buffer A), 80% ACN/0.02% HFBA/0.5% acetic acid (buffer B), 250 mM ammonium acetate/5% ACN/0.02% HFBA/0.5% acetic acid (buffer C), and 1.5 M ammonium acetate/5% ACN/0.02% HFBA/0.5% acetic acid (buffer D). The first step of 106 min consisted of a 100 min gradient from 0 to 80% buffer B and a 6 min hold at 80% buffer B. The next 6 steps (106 min each) are characterized by the following profile: 5 min of 100% buffer A, 2 min of x % buffer C, 5 min of 100% buffer A, a 3 min gradient from 0 to 10% buffer B, a 55 min gradient from 10 to 35% buffer B, a 20 min gradient from 35 to 50% buffer B, a 16 min gradient from 50 to 80% buffer B. The 2 min buffer C percentages (x) in steps 2-7 were as follows: 10, 20, 30, 40, 70, 90, and 100%. Step 8 consisted of the following profile: a 5 min 100% buffer A wash, a 20 min salt wash with 100% buffer D and a 100 min gradient from 0- 80% buffer B.

The HPLC column was directly coupled to a Finnigan LCQ ion trap mass spectrometer (Finnigan, Bremen, Germany) equipped with a nano-LC electrospray ionization source. Mass spectrometry in the MS-MS mode was performed according to the manufacturer's protocol. The identification of peptides was done by the SEQUEST algorithm against the swiss.fasta database.

### In silico Prediction of Potential Epitopes by TEPITOPE

Prediction of potential T cell epitopes was achieved by using the TEPITOPE algorithm. The following search criteria were applied: threshold (1-3% for best scoring and 4-6% for moderate scoring natural ligands), peptide length (15 amino acid residues) and promiscuity (predicted to bind to at least 6 out of 9 alleles). To determine the degree of promiscuity the following 9. alleles were chosen in agreement with their frequent occurrence in the Caucasian population: *HLA-DRB1***0101,* **0301,* **0401,* **0701,* **0801,* **1101,* **1305,* **1501* and *DRB5***0101.* Membrane-spanning domains and signal peptides were not included in the epitope search.

### T cell Activation Assay

The preparation of CD4⁺ T cells from fresh PBMCs was performed by negative selection using a CD4⁺ T cell isolation kit from Miltenyi Biotech (Auburn, CA, USA). The T cell population was >75% pure and >95% viable as judged by Trypan blue staining (Sigma-Aldrich). T cells were resuspended at 2 x 10⁶ cells/ml in AIM V medium (Gibco BRL, Rockville, MD). Dendritic cells (DCs) were differentiated from PBMCs as described and cultured in complete Macrophage-SFM medium (Gibco BRL, Rockville, MD). On day 4, immature DCs were stimulated with 10 µg/ml LPS (Sigma-Aldrich). On day 6, matured DCs were washed and resuspended in AIM V medium at 2 x 10⁵ cells/ml. For the co-culture, 0.1 ml CD4⁺ T cells (2 x 10⁵) and 0.1 ml autologous DCs (2 x 10⁴), both in AIM V medium, were mixed in a round-bottomed 96-well format plate. OKT3 mAb and Inflexal V^{®}were added to a final concentration of 20 µg/ml and 1 µg/ml, respectively. Synthetic peptides were added to a final concentration of 20 µM. Each antigen was tested in triplicate. On day 5 of the co-culture, 10 µM 5-bromo-2'-deoxyuridine (BrdU) (Roche, Basel, Switzerland) was added to each well. After 24 hrs incubation, cultures were harvested and processed according to the manufacturer's protocol. T cell proliferation of cultures without added antigen was used as reference with an average stimulation index (SI) set to 1.

For restimulation of T cells, immature DCs (2-3 x 10⁶/ml) were frozen at -70°C in 50% AB serum (Sigma-Aldrich), 40% RPMI and 10% DMSO (Sigma-Aldrich). At the time point of restimulation, DCs were defrosted, washed and cultivated for 2 days in the presence of 10 µg/ml LPS. On day 5 (1^{st} restimulation) or day 10 (2nd restimulation) of the DC/T cell co-culture, 0.1 ml AIM V medium was withdrawn from each sample well prior to adding 0.1 ml of defrosted, mature DCs (2 x 10⁴) in AIM V to the co-culture together with fresh protein or peptide antigen. IL-2 (Pharmingen, San Diego, CA) was added in a final concentration of 100 U/ml.

### Example 1

OKT3 was the first therapeutic antibody. It has been approved by the FDA in 1986. It is a CD3-specific mouse IgG2a antibody and widely used in the clinic as an immunosuppressive drug in transplantation (L. Chatenaud, 2003), type 1 diabetes (E. Masteller & J. Bluestone, 2002) and psoriasis (T. Udset et al., 2002). Despite the profound OKT3-induced immunosuppression, the occurrence of an anti-OKT3 response to the xenogeneic protein was one of the main drawbacks in early clinical trials promoting rapid clearance and neutralization of OKT3 (G. Goldstein, 1987). It has been reported that the incidence of immunogenicity is roughly 85% in studies involving OKT3-treated individuals (C. Pendley et al., 2003).

The strategy outlined in Figure 1 was used to identify peptide epitopes of OKT3, presented by dendritic cells displaying the HLA-DR genotype HLA-DRB1*0401/1302.

To identify HLA-DRB1*0401/1302-restricted OKT3 epitopes, dendritic cells, expressing the genotype HLA-DRB1*0401/1302 were differentiated from peripheral blood monocytes and cultured at a concentration of 0.5 x 10⁶ cells / ml. 5 x 10⁶ dendritic cells were exposed to the antibody OKT3 at a concentration of 20 µg/ml. At the same time, maturation of dendritic cells was induced by adding TNFα (10 ng/ml). As a control, the same amount of dendritic cells was cultured in the absence of OKT3, but in the presence of TNFα. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0401/1302-associated ligands revealed 3 OKT3- derived epitopes, represented by 7 peptide sequences derived from OKT3 (Table 1). Two of the epitopes were derived from the κ light chain, one epitope was located in the heavy chain. The three epitopes associated to the haplotypes DRB1*0401/1302 were found in at least 2 independent experiments.

Epitope #1 was represented by a 15- and 16-mer peptide, the 15-mer being derived from the constant part of the light chain region 99-113 (Table 1). Epitope #1 contains the anchor motif of the DRB1*1302-associated co-dominant DRB3 allele DRB3*0301 (F. Verreck et al. 1996):L-103 as P1, N-106 as P4, A-108 as P6 and A-111 as P9 anchor. The same anchor residues may confer binding to DRB1*0401, as indicated by the TEPITOPE algorithm (Figure 2). Epitope #1 was verified as a T cell epitope through its potency to induce proliferation of CD4+ T cells: Epitope #1 was stimulatory in context of dendritic cells that displayed the genotypes DRB1*0401 / *0701 (Figure 3B) and DRB1*0301 / *1501 (Fig. 3C). However, it was incapable of activating T cells in context of the genotype DRB1*1001 / *1201.

Epitope #2 was represented by 4 length variants: a 13-mer, a 14-mer , a15- mer and a 16-mer peptide. This epitope was also derived from the constant region of the light chain subunit (Table 1). Epitope #2 was predicted by the TEPITOPE algorithm in the context of DRB1*0401 (Figure 2) and contains the following anchor motif: W-147 as P1, D-150 as P4, S-152 as P6 anchor. In the T cell activation assay, epitope #2 stimulated proliferation of T cells in the context of the genotypes DRB1*0401 / *0701 (Figure 3B) and DRB1*0301 / *1501 (Fig. 3C). However, it did not stimulate T cells in context of the genotype DRB1*1001 / *1201. The homologous human sequence of epitope #2 was described in the context of the DRB1*0401 allele, extracted from an EBV-transformed B cell line (Friede et al., 1996).

Epitope #3 was represented by only one length variant: the 17-mer 194-210 was derived from the constant region of the OKT3 heavy chain (Table 1). Similar to epitope #1, epitope #3 contains the anchor motif of the DRB3 allele DRB3*0301: I-199 as P1, N-202 as P4, A-204 as P6 and A-207 as P9 anchor. Although the TEPITOPE algorithm did not predict epitope #3, neither for DRB1*0301, nor for *0401, *0701 or *1101 (Figure 2), epitope #3 activated T cells in the context of all 3 DRB1 genotypes tested (Figure 3). The same epitope has been described to be associated to the murine MHC class II molecule H2-A(s) (Rudenskyet al., 1992).

When the TEPITOPE algorithm was employed to predict epitopes of the OKT3 kappa light chain in the context of the genotype DRB1*0401/1302, only predictions for DRB1*0401 could be made because the other alleles are not covered by the algorithm (Figure 2). TEPITOPE predicted 11 epitopes in the kappa light chain, however, only two of them were among the naturally processed peptide epitopes, represented by epitopes #1 and #2 (Figure 2). Likewise, TEPITOPE predicted 13 epitopes in the OKT3 heavy chain, however, none of them covered epitope #3 (Figure 2).

### Example 2

The strategy outlined in Figure 1 was also used to identify peptide epitopes of OKT3, presented by dendritic cells displaying the HLA-DR genotype HLA-DRB1*0701/1601.

To identify HLA-DRB1*0701/1601-restricted OKT3 epitopes, dendritic cells, expressing the genotype HLA-DRB1*0701/1601 were differentiated from peripheral blood monocytes and cultured at a concentration of 0.5 x 10⁶ cells / ml. 5 x 10⁶ dendritic cells were exposed to the antibody OKT3 at a concentration of 20 µg/ml. At the same time, maturation of dendritic cells was induced by adding TNFα (10 ng/ml). As a control, the same amount of dendritic cells was cultured in the absence of OKT3, but in the presence of TNFα. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0701/1601-associated ligands revealed one OKT3- derived epitope, represented by 10 peptide sequences derived from OKT3 (Table 2). The epitope #4 was derived from the κ light chain and found in at least 2 independent experiments.

Epitope #4 was represented by a 10 length variants (15- 22-mers) peptide, the 15-mer being derived from the constant part of the light chain region 168-182 (Table 2). Epitope #4 contains the anchor motif of the DRB1*0701 allele: Y-172 as P1, S-175 as P4, T-177 as P6 and L-180 as P9 anchor. The same anchor residues may confer binding to DRB1*0441 and DRB1*1101, as indicated by the TEPITOPE algorithm (Figure 2). Epitope #1 was verified as a T cell epitope through its potency to induce proliferation of CD4+ T cells: Epitope #4 was stimulatory in context of dendritic cells that displayed the genotypes DRB1*0401 / *0701 (Figure 3B) and DRB1*0301 / *1501 (Fig. 3C). However, it was incapable of activating T cells in context of the genotype DRB3*1001 / *1201.

Epitope #4 was recently described in the bovine system, extracted from blood mononuclear cells and presented by the bovine allele DRB3*2703 (Sharif et al., 2002).

When the TEPITOPE algorithm was employed to predict epitopes of the OKT3 kappa light chain in the context of the genotype DRB1*0701 / *1601 only predictions for DRB1*0401 could be made because the DRB1*1601 allele is not covered by the algorithm (Figure 2). TEPITOPE predicted 5 epitopes in the kappa light chain, however, only one of them was among the naturally processed peptide epitopes, represented by epitope #4 (Figure 2). Likewise, TEPITOPE predicted 8 epitopes in the OKT3 heavy chain, however, none of them was supported by the analysis of naturally occurring peptides (Figure 2).

### Example 3

The strategy outlined in Figure 1 was used to identify peptide epitopes of OKT3, as recognized by T cells restricted by the HLA-DR genotypes HLA-DRB1*1101/1202.

To identify HLA-DRB1*1101/1202-restricted OKT3 epitopes, dendritic cells, expressing the genotype HLA-DRB1*1101/1202 were differentiated from peripheral blood monocytes and cultured at a concentration of 0.5 x 10⁶ cells / ml. 5 x 10⁶ dendritic cells were exposed to the antibody OKT3 at a concentration of 20 µg/ml. At the same time, maturation of dendritic cells was induced by adding TNFα (10 ng/ml). As a control, the same amount of dendritic cells was cultured in the absence of OKT3, but in the presence of TNFα. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*1101/1202-associated ligands revealed 2 OKT3- derived epitopes, #1 and #3, represented by 6 peptide sequences derived from OKT3 (Table 3). One epitope was derived from the κ light chain the other epitope was located in the heavy chain. The two epitopes associated to the haplotypes DRB1*1101/1202 were found in at least 2 independent experiments.

Epitope #1 was represented by the same 15- and 16-mer peptide that has been described above in the context of the genotypes DRB1*0401 / *1302 (cf. Tables 1 and 3). Epitope #1 contains the anchor motif of the DRB1*1101 allele: L-103 as P1, A-108 as P6 and A-111 as P9 anchor. Epitope #1 was verified as a T cell epitope through its potency to induce proliferation of CD4+ T cells: Epitope #1 was stimulatory in context of dendritic cells that displayed the genotypes DRB1*0401 / *0701 (Figure 3B) and DRB1*0301 / *1501 (Fig. 3C). However, it was incapable of activating T cells in context of the genotype DRB1*1001 / *1201.

Epitope #3, derived from the constant region of the OKT3 heavy chain (Tables 1,3), was represented by 4 length variants: the 14-mer 194-207, the 15-mer 194-208, the 17-mer 194-210 and the 18-mer 194-211 (Table 3). Although the TEPITOPE algorithm did not predict epitope #3, neither for DRB1*1101, nor for *1202 (Figure 2), epitope #3 activated T cells in the context of all 3 DRB1 genotypes tested (Figure 3).

When the TEPITOPE algorithm was employed to predict epitopes of the OKT3 kappa light chain in the context of the genotype DRB1*1101/1202, only predictions for DRB1*1101 could be made because the other alleles are not covered by the algorithm (Figure 2). TEPITOPE predicted 5 epitopes in the kappa light chain, however, only one epitope was among the naturally processed peptide epitopes, represented by epitope #1 (Figure 2). Likewise, TEPITOPE predicted 9 epitopes in the OKT3 heavy chain, however, none of them covered epitope #3 (Figure 2).

### Example 4

The strategy outlined in Figure 1 was also used to identify peptide epitopes of OKT3, presented by dendritic cells displaying the HLA-DR genotype HLA-DRB1*0301/0401.

To identify HLA-DRB1*0301/0401-restricted OKT3 epitopes, dendritic cells, expressing the genotype HLA-DRB1*0301/0401 were differentiated from peripheral blood monocytes and cultured at a concentration of 0.5 x 10⁶ cells / ml. 5 x 10⁶ dendritic cells were exposed to the antibody OKT3 at a concentration of 20 µg/ml. At the same time, maturation of dendritic cells was induced by adding TNFα (10 ng/ml). As a control, the same amount of dendritic cells was cultured in the absence of OKT3, but in the presence of TNFα. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0301/0401-associated ligands revealed one OKT3- derived epitope, represented by 1 peptide sequence derived from OKT3 (Table 2). The epitope #2 was derived from the κ light chain and found in at least 2 independent experiments.

Epitope #2 was represented by the 17-mer peptide 143-159 derived from the constant part of the light chain (Table 4). As described above (example 1), epitope #2 contains the anchor motif of the DRB1*0401 allele: W-147 as P1, D-150 as P4, S-152 as P6 anchor. The same anchor residues may confer binding to DRB1*0301, as indicated by the TEPITOPE algorithm (Figure 2). Epitope #2 was verified as a T cell epitope through its potency to induce proliferation of CD4+ T cells: Epitope #2 was stimulatory in context of dendritic cells that displayed the genotypes DRB1*0401 / *0701 (Figure 3B) and DRB1*0301 / *1501 (Fig. 3C). However, epitope #2 was incapable of activating T cells in context of the genotype DRB1*1001 / *1201.

The TEPITOPE algorithm was employed to predict epitopes of the OKT3 kappa light chain in the context of the genotype DRB1*0301 / *0401 (Figure 2). TEPITOPE predicted 12 epitopes in the kappa light chain, however, only one of them was among the naturally processed peptide epitopes, represented by epitope #2 (Figure 2). Likewise, TEPITOPE predicted 18 epitopes in the OKT3 heavy chain, however, none of them was supported by the analysis of naturally occurring peptides (Figure 2).

### Example 5

Interferon-beta (IFN-β) is currently the first-line therapy for treatment of multiple sclerosis (Deisenhammer et al., 2000). Three different IFN-β formulations are currently marketed: Avonex, Rebif (both IFN-β-1a) and Betaseron (IFN-β-1b). Thereof Betaseron was the first one on the market, being approved by FDA in 1993 under accelerated approval regulations. In contrast to Avonex and Rebif, Betaseron is known to be exceptionally immunogenic. After treatment with Betaseron as much as 28-47 % of patients produce anti- IFN-β neutralizing antibodies, while only 2-6% of Avonex-treated patients show neutralizing anti-drug antibodies (Deisenhammer et al., 2000; Bertolotto et al., 2004). In this context it is important to mention that Avonex and Rebif are expressed in Chinese hamster ovary cells as glycosylated proteins with the natural amino acid sequence, while Betaseron is expressed in E. coli in a non-glycosylated form with a Met-1 deletion and a Cys-17 to Ser point-mutation (Mark et al., 1984; Holliday and Benfield, 1997). So far it is unclear if these differences are responsible for the varying immunogenicity.

The strategy outlined in Figure 1 was used to identify peptide epitopes of IFN-β-1b, presented by dendritic cells displaying the HLA-DR genotype HLA-DRB1*0101/0701.

To identify HLA-DRB1*0101/0701-restricted IFN-β-1b epitopes, dendritic cells, expressing the genotype HLA-DRB1*0101/0701 were differentiated from peripheral blood monocytes and cultured at a concentration of 0.5 x 10⁶ cells / ml. 5 x 10⁶ dendritic cells were exposed to IFN-β-1b at a concentration of 20 µg/ml. At the same time, maturation of dendritic cells was induced by adding lipopolysaccharide (LPS) at a concentration of 1 µg/ml). As a control, the same amount of dendritic cells was cultured in the absence of IFN-β-1b, but in the presence of LPS. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0101/0701-associated ligands revealed one IFN-β-1b -derived epitope, #5, represented by 3 peptide sequences derived from IFN-β-1b (Table 5). The epitope #5 associated to the genotype DRB1*0101/0701 was also found in the context of the genotype DRB1*0101/1401 (cf. Table 8).

Epitope #5 was represented by a 13-mer, a 16-mer and a 17-mer peptide, the 13-mer being derived from the protein region 44-60 (Table 5). Epitope #5 contains the following anchor motif: F-49 as P1, E-52 as P4, A-54 as P6 and T-57 as P9 anchor. Consistently, in in vitro binding assays it has been shown that a 15-mer peptide containing epitope #5 has strong bin dig capabilities for the HLA allele DRB1*0101 (Tangri et al., 2005).

### Example 6

The strategy outlined in Figure 1 was used to identify peptide epitopes of IFN-β-1b, presented by dendritic cells displaying the HLA-DR genotype HLA-DRB1*L101/1404.

To identify HLA-DRB1*1101/1404-restricted IFN-β-1b epitopes, dendritic cells, expressing the genotype HLA-DRB1*1101/1404 were differentiated from peripheral blood monocytes and cultured at a concentration of 0.5 x 10⁶ cells / ml. 5 x 10⁶ dendritic cells were exposed to IFN-β-1b at a concentration of 20 µg/ml. At the same time, maturation of dendritic cells was induced by adding lipopolysaccharide (LPS) at a concentration of 1 µg/ml). As a control, the same amount of dendritic cells was cultured in the absence of IFN-β-1b, but in the presence of LPS. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*1101/1404-associated ligands revealed 2 IFN-β-1b -derived epitopes, #6 and #7, represented by 24 peptide sequences derived from IFN-β-1b (Table 6). Epitope #6 was also found in the context of the genotype DRB1*0801 (Table 7). Epitope #7 was also found in the context of genotype DRB1*0801 (Table 7), DRB1*0101/14 (Table 8) and DRB1*1303/1501 (Table 9).

Epitope #6 was represented by 22 length variants (11-19-mer), the 11-mer being derived from the protein region 89-99 (Table 6). Epitope #6 contains the following anchor motif: Y-91 as P1, I-94 as P4, H-96 as P6 and T-99 as P9 anchor. These anchor residues may confer binding to the HLA alleles DRB1*1101 and DRB1*0801, as predicted by the TEPITOPE algorithm. Although a 15-mer peptide containing the epitope #6 has been shown to bind to DRB1*0701 there was no evidence for T cell activation in this HLA context (Barbosa et al., 2005).

Epitope #7 was represented by a 13-mer and a 15-mer peptide, the 13-mer being from the protein region 149-161 (Table 6). Epitope #7 contains the following anchor motif: F-153 as P1, I-156 as P4, R-158 as P6 and G-161 as P9 anchor. A 15-mer peptide containing the epitope #7 has also been shown to be a promiscuous binder with very strong binding capabilities for the HLA alleles DRB1*0101, DRB1*1101 and DRB1*1501 (Tangri et al., 2005). Furthermore is has been described that a peptide pool containing the epitope #7 induces T cell activation in a DRB1*0701 background (Barbosa et al., 2005).

### Example 7

The strategy outlined in Figure 1 was used to identify peptide epitopes of IFN-β-1b, presented by dendritic cells displaying the HLA-DR genotype HLA-DRB1*0801/0801.

To identify HLA-DRB1*0801/0801-restricted IFN-β-1b epitopes, dendritic cells, expressing the genotype HLA-DRB1*0801/0801 were differentiated from peripheral blood monocytes and cultured at a concentration of 0.5 x 10⁶ cells / ml. 5 x 10⁶ dendritic cells were exposed to IFN-β-1b at a concentration of 20 µg/ml. At the same time, maturation of dendritic cells was induced by adding lipopolysaccharide (LPS) at a concentration of 1 µg/ml). As a control, the same amount of dendritic cells was cultured in the absence of IFN-β-1b, but in the presence of LPS. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0801/0801-associated ligands revealed 2 IFN-β-1b -derived epitopes, represented by 22 peptide sequences derived from IFN-β-1b (Table 7). The two epitopes associated to the genotype DRB1*0801/0801 were found in at least 2 independent experiments.

Epitope #6 was represented by 17 length variants (11-18-mer), the 11-mer being derived from the protein region 89-99 (Table 6). As described above for DRB1*1101/1404 epitope #6 contains the following anchor motif: Y-91 as P1, I-94 as P4, H-96 as P6 and T-99 as P9 anchor. These anchor residues may confer binding to the HLA alleles DRB1*1101 and DRB1*0801, as predicted by the TEPITOPE algorithm.

Epitope #7 was represented by the following 5 length variants: The 11-mer 151-161, the 13-mer 149-161, the 14-mer 149-162, the 14-mer 148-161 and the 15-mer 147-161 (Table 7). Epitope #7 contains the following anchor motif: F-153 as P1, I-156 as P4, R-158 as P6 and G-161 as P9 anchor.

### Example 8

The strategy outlined in Figure 1 was used to identify peptide epitopes of IFN-β-1b, presented by dendritic cells displaying the HLA-DR genotype HLA-DRB1*0101/1401.

To identify HLA-DRB1*0101/1401-restricted IFN-β-1b epitopes, dendritic cells, expressing the genotype HLA-DRB1*0101/1401 were differentiated from peripheral blood monocytes and cultured at a concentration of 0.5 x 10⁶ cells / ml. 5 x 10⁶ dendritic cells were exposed to IFN-β-1b at a concentration of 20 µg/ml. At the same time, maturation of dendritic cells was induced by adding lipopolysaccharide (LPS) at a concentration of 1 µg/ml). As a control, the same amount of dendritic cells was cultured in the absence of IFN-β-1b, but in the presence of LPS. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*0101/1401-associated ligands revealed 2 IFN-β-1b -derived epitopes, represented by 9 peptide sequences derived from IFN-β-1b (Table 8). The two epitopes associated to the genotype DRB1*0101/1401 were found in at least 2 independent experiments.

Epitope #5 was represented by 7 length variants: The 15-mer 46-60, the 16-mer 45-60, the 17-mer 44-60, the 18-mer 43-60, the 19-mer 43-61, the 19-mer 42-60 and the 22-mer 39-60 (Table 8). Epitope #5 contains the following anchor motif: F-49 as P1, E-52 as P4, A-54 as P6 and T-57 as P9 anchor.

Epitope #7 was represented by a 13-mer and a 15-mer peptide, the 13-mer being from the protein region 149-161 (Table 8). Epitope #7 contains the following anchor motif: F-153 as P1, I-156 as P4, R-158 as P6 and G-161 as P9 anchor.

### Example 9

The strategy outlined in Figure 1 was used to identify peptide epitopes of IFN-β-1b, presented by dendritic cells displaying the HLA-DR genotype HLA-DRB1*1303/1501.

To identify HLA-DRB1*1303/1501-restricted IFN-β-1b epitopes, dendritic cells, expressing the genotype HLA-DRB1*1303/1501 were differentiated from peripheral blood monocytes and cultured at a concentration of 0.5 x 10⁶ cells / ml. 5 x 10⁶ dendritic cells were exposed to IFN-β-1b at a concentration of 20 µg/ml. At the same time, maturation of dendritic cells was induced by adding lipopolysaccharide (LPS) at a concentration of 1 µg/ml). As a control, the same amount of dendritic cells was cultured in the absence of IFN-β-1b, but in the presence of LPS. After an incubation period of 24 hrs, both sets of dendritic cells were lysed in detergent TX-100 and HLA-DR molecules were precipitated by using the anti-HLA-DR mAb L243 immobilized to sepharose beads. HLA-DR associated peptides were eluted with 0.1% TFA and analyzed by 2D-LS/MS-MS.

Sequence analysis of HLA-DRB1*1303/1501-associated ligands revealed 1 IFN-β-1b -derived epitope, represented by three peptide sequences derived from IFN-β-1b (Table 9). The epitope associated to the genotype DRB1*1303/1501 was found in at least 2 independent experiments.

Epitope #7 was represented by the 13-mer 149-161, the 16-mer 148-161 and the 17-mer 147-161 (Table 9). Epitope #7 contains the following anchor motif: F-153 as P1, I-156 as P4, R-158 as P6 and G-161 as P9 anchor.

**Table 1.**

| OKT-3 (Orthoclone) epitopes associated to the genotype HLA-DRB1*0401/*1302. | | | | |
|---|---|---|---|---|
| | | | | |

| Epitope no. | Peptide sequence | OKT-3 subunit | OKT-3 region | SEQ. ID. NO |
|---|---|---|---|---|
| | | | | |
| | | | | |
| | | | | |
| #1 | SGTKLEINRADTAPT | κ light chain | 99-113 | 3 |
| | GSGTKLEINRADTAPT | | 98-113 | 4 |
| | | | | |
| | | | | |
| #2 | VKWKIDGSERQNG | κ light chain | 145-157 | 5 |
| | NVKWKIDGSERQNG | | 144-157 | 6 |
| | INVKWKIDGSERQNG | | 143-157 | 7 |
| | INVKWKIDGSERQNGV | | 143-158 | 8 |
| | | | | |
| | | | | |
| #3 | WPSQSITCNVAHPASST | heavy chain | 194-210 | 9 |
| | | | | |
| | | | | |

**Table 2.**

| OKT-3 (Orthoclone) epitopes associated to the genotype HLA-DRB1*0701/*1601. | | | | |
|---|---|---|---|---|
| | | | | |

| Epitope no. | Peptide sequence | OKT-3 subunit | OKT-3 region | SEQ.ID. NO |
|---|---|---|---|---|
| | | | | |
| | | | | |
| | | | | |
| #4 | KDSTYSMSSTLTLTK | κ light chain | 168-182 | 10 |
| | KDSTYSMSSTLTLTKD | | 168-183 | 11 |
| | KDSTYSMSSTLTLTKDE | | 168-184 | 12 |
| | SKDSTYSMSSTLTLTKD | | 167-183 | 13 |
| | SKDSTYSMSSTLTLTKDE | | 167-184 | 14 |
| | DSKDSTYSMSSTLTLTKD | | 166-183 | 15 |
| | DSKDSTYSMSSTLTLTKDE | | 166-184 | 16 |
| | DQDSKDSTYSMSSTLTLTKD | | 164-183 | 17 |
| | DQDSKDSTYSMSSTLTLTKDE | | 164-184 | 18 |
| | TDQDSKDSTYSMSSTLTLTKDE | | 163-184 | 19 |
| | | | | |
| | | | | |

**Table 3.**

| OKT-3 (Orthoclone) epitopes associated to the genotype HLA-DRB1*1101/*1202. | | | | |
|---|---|---|---|---|
| | | | | |

| Epitope no. | Peptide sequence | OKT-3 subunit | OKT-3 region | SEQ. ID. NO |
|---|---|---|---|---|
| | | | | |
| | | | | |
| | | | | |
| #1 | SGTKLEINRADTAPT | κ light chain | 99-113 | 20 |
| | GSGTKLEINRADTAPT | | 98-113 | 21 |
| | | | | |
| | | | | |
| #3 | WPSQSITCNVAHPA | heavy chain | 194-207 | 22 |
| | WPSQSITCNVAHPAS | | 194-208 | 23 |
| | WPSQSITCNVAHPASST | | 194-210 | 24 |
| | WPSQSITCNVAHPASSTK | | 194-211 | 25 |
| | | | | |
| | | | | |

**Table 4.**

| OKT-3 (Orthoclone) epitopes associated to the genotype HLA-DRB1*0301/*0401. | | | | |
|---|---|---|---|---|
| Epitope no. | Peptide sequence | OKT-3 subunit | OKT-3 region | SEQ.ID. NO |
| | | | | |
| | | | | |
| | | | | |
| #2 | INVKWKIDGSERQNGVL | κ light chain | 143-159 | 26 |
| | | | | |
| | | | | |

**Table 5.**

| Interferon-β-1b epitopes associated to the genotype HLA-DRB1*0101/*0701. | | | |
|---|---|---|---|
| | | | |

| Epitope no. | Peptide sequence | IFN-β-1b region | SEQ. ID. NO |
|---|---|---|---|
| | | | |
| | | | |
| | | | |
| #5 | QFQKEDAALTIYE | 48-60 | 27 |
| | QLQQFQKEDAALTIYE | 45-60 | 28 |
| | KQLQQFQKEDAALTIYE | 44-60 | 29 |
| | | | |

**Table 6.**

| Interferon-β-1b epitopes associated to the genotype HLA-DRB1*1101/*1404. | | | |
|---|---|---|---|
| Epitope no. | Peptide sequence | IFN-β-1b region | SEQ. ID. NO |
| | | | |
| | | | |
| | | | |
| #6 | NVYHQINHLKT | 89-99 | 30 |
| | NVYHQINHLKTV | 89-100 | 31 |
| | NVYHQINHLKTVL | 89-101 | 32 |
| | NVYHQINHLKTVLE | 99-102 | 33 |
| | NVYHQINHLKTVLEE | 89-103 | 34 |
| | NVYHQINHLKTVLEEK | 89-104 | 35 |
| | ANVYHQINHLKT | 88-99 | 36 |
| | ANVYHQINHLKTV | 88-100 | 37 |
| | ANVYHQINHLKTVL | 88-101 | 38 |
| | ANVYHQINHLKTVLE | 88102 | 39 |
| | ANVYHQINHLKTVLEE | 88-103 | 40 |
| | ANVYHQINHLKTVLEEK | 88-104 | 41 |
| | LANVYHQINHLKTV | 87-100 | 42 |
| | LANVYHQINHLKTVL | 87-101 | 43 |
| | LANVYHQINHLKTVLE | 87-102 | 44 |
| | LANVYHQINHLKTVLEE | 87-103 | 45 |
| | LLANVYHQINHLKTVL | 86-101 | 46 |
| | LLANVYHQINHLKTVLE | 86-102 | 47 |
| | LLANVYHQINHLKTVLEE | 86-103 | 48 |
| | LLANVYHQINHLKTVLEEK | 86-104 | 49 |
| | NLLANVYHQINHLKTVLE | 85-102 | 50 |
| | NLLANVYHQINHLKTVLEE | 85-103 | 51 |
| | | | |
| | | | |
| #7 | ILRNFYFINRLTG | 149-161 | 52 |
| | VEILRNFYFINRLTG | 147-161 | 53 |
| | | | |

**Table 7.**

| Interferon-β-1b epitopes associated to the genotype HLA-DRB1*0801/*0801. | | | |
|---|---|---|---|
| | | | |

| Epitope no. | Peptide sequence | IFN-β-1b region | SEQ. ID. NO |
|---|---|---|---|
| | | | |
| | | | |
| | | | |
| #6 | NVYHQINHLKT | 89-99 | 54 |
| | NVYHQINHLKTV | 89-100 | 55 |
| | NVYHQINHLKTVL | 89-101 | 56 |
| | NVYHQINHLKTVLE | 99-102 | 57 |
| | NVYHQINHLKTVLEE | 89-103 | 58 |
| | ANVYHQINHLKT | 88-99 | 59 |
| | ANVYHQINHLKTV | 88-100 | 60 |
| | ANVYHQINHLKTVL | 88-101 | 61 |
| | ANVYHQINHLKTVLE | 88-102 | 62 |
| | LANVYHQINHLKT | 87-99 | 63 |
| | LANVYHQINHLKTV | 87-100 | 64 |
| | LANVYHQINHLKTVL | 87-101 | 65 |
| | LANVYHQINHLKTVLE | 87-102 | 66 |
| | LLANVYHQINHLKT | 86-99 | 67 |
| | LLANVYHQINHLKTVLE | 86-102 | 68 |
| | NLLANVYHQINHLKT | 85-99 | 69 |
| | NLLANVYHQINHLKTVLE | 85-102 | 70 |
| | | | |
| #7 | RNFYFINRLTG | 151-161 | 71 |
| | ILRNFYFINRLTG | 149-161 | 72 |
| | ILRNFYFINRLTGY | 149-162 | 73 |
| | EILRNFYFINRLTG | 148-161 | 74 |
| | VEILRNFYFINRLTG | 147-161 | 75 |
| | | | |

**Table 8.**

| Interferon-β-1b epitopes associated to the genotype HLA-DRB1*0101/*1401. | | | |
|---|---|---|---|
| | | | |

| Epitope no. | Peptide sequence | IFN-β-1b region | SEQ. ID. NO |
|---|---|---|---|
| | | | |
| | | | |
| | | | |
| #5 | LQQFQKEDAALTIYE | 46-60 | 76 |
| | QLQQFQKEDAALTIYE | 45-60 | 77 |
| | KQLQQFQKEDAALTIYE | 44-60 | 78 |
| | IKQLQQFQKEDAALTIYE | 43-60 | 79 |
| | IKQLQQFQKEDAALTIYEM | 43-61 | 80 |
| | EIKQLQQFQKEDAALTIYE | 42-60 | 81 |
| | IPEEIKQLQQFQKEDAALTIYE | 39-60 | 82 |
| | | | |
| | | | |
| #7 | ILRNFYFINRLTG | 149-161 | 83 |
| | VEILRNFYFINRLTG | 147-161 | 84 |
| | | | |

**Table 9.**

| Interferon-β-1b epitopes associated to the genotype HLA-DRB1*1303/*1501. | | | |
|---|---|---|---|
| | | | |

| Epitope no. | Peptide sequence | IFN-β-1b region | SEQ. ID. NO |
|---|---|---|---|
| | | | |
| | | | |
| | | | |
| #7 | ILRNFYFINRLTG | 149-161 | 85 |
| | EILRNFYFINRLTG | 148-161 | 86 |
| | VEILRNFYFINRLTG | 147-161 | 87 |

## Claims

1. A method for decreasing the immunogenicity of a therapeutic polypeptide comprising
a) identifying the immunogenic peptides of the therapeutic polypeptide comprising
i) providing cells expressing MHC II molecules in a total amount of 0.1 to 5 µg molecules,
ii) contacting the cells from (i) with a source of immunogenic peptides,
iii) isolating MHC II molecule-immunogenic peptide complexes from the cells,
iv) eluting the associated peptides from the MHC II molecules.
v) identifying the immunogenic peptides
vi) verifying the identified immunogenic peptides as epitopes
b) modifying the corresponding epitopes of the polypeptide so that the binding of MHC II molecules is reduced or abolished
c) thereby creating a modified polypeptide with reduced or no immunogenicity potential.

2. The method according to claim 1, wherein the MHC II expressing cells are dendritic cells.

3. The method according to claim 2, wherein the dendritic cells are exposed to a potential source of immunogen as immature dendritic cells at the same time as they are induced to mature to dendritic cells.

4. The method according to claims 1 to 3, wherein the source of potential immunogen belongs to the group comprising polypeptides including therapeutic polypeptides as cytokines, chemokines, growth factors, antibodies, enzymes, structural proteins, hormones or a fragment thereof.

5. The method according to claims 1 to 4, wherein the complexes of MHC II molecules with immunogenic peptides are isolated from the cells with methods comprising solubilization of the cells with a detergent and sequestration of the complexes of MHC II molecules with immunogenic peptides by immunoprecipitation or immunoaffinity chromatography.

6. The method according to claims 1 to 5, wherein the sequestered complexes of MHCII molecules with immunogenic peptides are washed with water in an ultrafiltration tube before eluting the peptides.

7. The method according to claims 1 to 6, wherein the immunogenic peptides are eluted from the MHCII molecules using diluted acid.

8. The method according to claims 1 to 7, wherein the isolated immunogenic peptides of (a)(iv) are fractionated and sequenced.

9. The method according to claim 8, wherein the isolated immunogenic peptides are fractionated and sequenced by methods comprising liquid chromatography and mass spectrometry.

10. The method according to claims 1 to 9, wherein the isolated immunogenic peptides are identified by comparing the peptide identified from cells which have been contacted with a source of potential immunogen with those, which have been identified from cells which have not been contacted with that source.

11. The method according to claims 1 to 10, wherein the immunogenic peptides are naturally-processed immunogenic peptides.

## Patentansprüche

1. Verfahren zur Verringerung der Immunogenität eines therapeutischen Polypeptids, umfassend
a) das Identifizieren des immunogenen Peptids des therapeutischen Polypeptids, umfassend
i) das Bereitstellen von Zellen, die MHC 11-Moleküle in einer Gesamtmenge von 0,1 bis 5 µg Molekülen exprimieren,
ii) das Kontaktieren der Zellen aus (i) mit einer Quelle immunogener Peptide,
iii) das Isolieren MHC II-Molekül-immunogener Peptidkomplexe aus den Zellen,
iv) das Eluieren der assoziierten Peptide aus den MHC II-Molekülen,
v) das Identifizieren der immunogenen Peptide,
vi) das Verifizieren der identifizierten immunogenen Peptide als Epitope,
b) das Modifizieren der entsprechenden Epitope des Polypeptids, so dass die Bindung der MHC II-Moleküle verringert oder aufgehoben wird,
c) **dadurch** das Erzeugen eines modifizierten Polypeptids mit verringertem oder ohne Immunogenitätspotential.

2. Verfahren nach Anspruch 1, wobei die MHC II exprimierenden Zellen dendritische Zellen sind.

3. Verfahren nach Anspruch 2, wobei die dendritischen Zellen einer potentiellen Immunogenquelle als unreife dendritische Zellen zur selben Zeit ausgesetzt werden, zu der ihr Reifen zu dendritischen Zellen induziert wird.

4. Verfahren nach den Ansprüche 1 bis 3, wobei die Quelle des potentiellen Immunogens zu der Gruppe gehört, umfassend Polypeptide, einsehließlich therapeutische Polypeptide wie Cytokine, Chemokine, Wachstumsfaktoren, Antikörper, Enzyme, Strukturproteine, Hormone oder ein Fragment davon.

5. Verfahren nach den Ansprüche 1 bis 4, wobei die Komplexe von MHC 11-Molekülen mit immunogenen Peptiden aus den Zellen mittels Verfahren, umfassend das Solubilisieren der Zellen mit einem Detergens und Sequestrieren der Komplexe von MHC II-Molekülen mit immunogenen Peptiden durch Immunfällung oder Immunaffinitätschromatographie, isoliert werden.

6. Verfahren nach den Ansprüchen 1 bis 5, wobei die sequestrierten Komplexe von MHC II-Molekülen mit immunogenen Peptiden mit Wasser in einem Ultrafiltrationsröhrchen gewaschen werden, bevor die Peptide eluiert werden.

7. Verfahren nach den Ansprüchen 1 bis 6, wobei die immunogenen Peptide aus den MHC II-Molekülen unter Verwendung verdünnter Säure eluiert werden.

8. Verfahren nach den Ansprüchen 1 bis 7, wobei die isolierten immunogenen Peptide von (a)(iv) fraktioniert und sequenziert werden.

9. Verfahren nach Anspruch 8, wobei die isolierten immunogenen Peptide durch Verfahren, umfassend Flüssigchromatographie und Massenspektrometrie, fraktioniert und sequenziert werden.

10. Verfahren nach den Ansprüchen 1 bis 9, wobei die isolierten immunogenen Peptide durch Vergleichen des Peptids, das aus den Zellen identifiziert wurde, die mit einer Quelle eines potentiellen Immunogens kontaktiert wurden, mit dem, das aus Zellen identifiziert wurde, die nicht mit dieser Quelle kontaktiert wurden, identifiziert werden.

11. Verfahren nach den Ansprüchen 1 bis 10, wobei die immunogenen Peptide natürlich prozessierte immunogene Peptide sind.

## Revendications

1. Procédé pour réduire l'immunogénicité d'un polypeptide thérapeutique, comprenant
a) l'identification des peptides immunogènes du polypeptide thérapeutique, comprenant:
i) la fourniture de cellules exprimant des molécules du CMH II en une quantité totale de 0,1 à 5 µg de molécules,
ii) la mise en contact des cellules de (i) avec une source de peptides immunogènes,
iii) l'isolement de complexes molécule de CMH II - peptide immunogène à partir des cellules,
iv) l'élution des peptides associés à partir des molécules de CMH II,
v) l'identification des peptides immunogènes,
vi) la vérification des peptides immunogènes identifiés en tant qu'épitopes,
b) la modification des épitopes correspondants du polypeptide de façon que la liaison des molécules du CMH II soit réduite ou supprimée,
c) et de ce fait la création d'un polypeptide modifié ayant un potentiel d'immunogénicité réduit ou nul.

2. Procédé selon la revendication 1, dans lequel les cellules exprimant le CMH II sont des cellules dendritiques.

3. Procédé selon la revendication 2, dans lequel on expose les cellules dendritiques à une source potentielle d'immunogène sous forme de cellules dendritiques immatures en même temps que l'on induit leur maturation en cellules dendritiques.

4. Procédé selon les revendications 1 à 3, dans lequel la source d'immunogène potentiel appartient au groupe comprenant des polypeptides incluant des polypeptides thérapeutiques comme des cytokines, des chimiokines, des facteurs de croissance, des anticorps, des enzymes, des protéines structurelles, des hormones ou leurs fragments.

5. Procédé selon les revendications 1 à 4, dans lequel on isole les complexes de molécules du CMH II avec des peptides immunogènes à partir des cellules par des procédés comprenant la solubilisation des cellules avec un détergent et la séquestration des complexes de molécules du CMH II avec des peptides immunogènes par immunoprécipitation ou chromatographie par immunoaffinité.

6. Procédé selon les revendications 1 à 5, dans lequel on lave les complexes de molécules du CMH II avec des peptides immunogènes séquestrés avec de l'eau dans un tube d'ultrafiltration avant d'éluer les peptides.

7. Procédé selon les revendications 1 à 6, dans lequel on élue les peptides immunogènes à partir des molécules du CMH II à l'aide d'un acide dilué.

8. Procédé selon les revendications 1 à 7, dans lequel on fractionne et on séquence les peptides immunogènes isolés de (a)(iv).

9. Procédé selon la revendication 8, dans lequel on fractionne et on séquence les peptides immunogènes isolés par des procédés comprenant la chromatographie liquide et la spectrométrie de masse.

10. Procédé selon les revendications 1 à 9, dans lequel on identifie les peptides immunogènes isolés en comparant le peptide identifié à partir de cellules qui ont été mises en contact avec une source d'immunogène potentiel avec ceux qui ont été identifiés à partir de cellules qui n'ont pas été mises en contact avec cette source.

11. Procédé selon les revendications 1 à 10, dans lequel les peptides immunogènes sont des peptides immunogènes traités de manière naturelle.


### SEQUENCE LISTING

<110> F. Hoffmann-La Roche AG
<120> Method for the identification of epitopes related to immunogenicity in biopharmaceuticals
<130> 23097
<160> 87
<170> Patent In version 3.3
<210> 1
   <211> 213
   <212> PRT
   <213> Mus musculus
<220>
   <221> OKT3 light chain (kappa)
   <222> (1)..(213)
<400> 1
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Tyr Met
   20 25 30
   Asn Trp Tyr Gln Gln Lys Ser Gly Thr Ser Pro Lys Arg Trp Ile Tyr
   35 40 45
   Asp Thr Ser Lys Leu Ala Ser Gly Val Pro Ala His Phe Arg Gly Ser
   50 55 60
   Gly Ser Gly Thr Ser Tyr Ser Leu Thr Ile Ser Gly Met Glu Ala Glu
   65 70 75 80
   Asp Ala Ala Thr Tyr Tyr Cys Gln Gln Trp Ser Ser Asn Pro Phe Thr
   85 90 95
   Phe Gly Ser Gly Thr Lys Leu Glu Ile Asn Arg Ala Asp Thr Ala Pro
   100 105 110
   Thr Val Ser Ile Phe Pro Pro Ser Ser Glu Gln Leu Thr Ser Gly Gly
   115 120 125
   Ala Ser Val Val Cys Phe Leu Asn Asn Phe Tyr Pro Lys Asp Ile Asn
   130 135 140
   Val Lys Trp Lys Ile Asp Gly Ser Glu Arg Gln Asn Gly Val Leu Asn
   145 150 155 160
   Ser Trp Thr Asp Gln Asp Ser Lys Asp Ser Thr Tyr Ser Met Ser Ser
   165 170 175
   Thr Leu Thr Leu Thr Lys Asp Glu Tyr Glu Arg His Asn Ser Tyr Thr
   180 185 190
   Cys Glu Ala Thr His Lys Thr Ser Thr Ser Pro Ile Val Lys Ser Phe
   195 200 205
   Asn Arg Asn Glu Cys
   210
<210> 2
   <211> 449
   <212> PRT
   <213> Mus musculus
<220>
   <221> OKT3 heavy chain (IgG2a)
   <222> (1)..(449)
<400> 2
   Gln Val Gln Leu Gln Gln Ser Gly Ala Glu Leu Ala Arg Pro Gly Ala
   1 5 10 15
   Ser Val Lys Met Ser Cys Lys Ala Ser Gly Tyr Thr Phe Thr Arg Tyr
   20 25 30
   Thr Met His Trp Val Lys Gln Arg Pro Gly Gln Gly Leu Glu Trp Ile
   35 40 45
   Gly Tyr Ile Asn Pro Ser Arg Gly Tyr Thr Asn Tyr Asn Gln Lys Phe
   50 55 60
   Lys Asp Lys Ala Thr Leu Thr Thr Asp Lys Ser Ser Ser Thr Ala Tyr
   65 70 75 80
   Met Gln Leu Ser Ser Leu Thr Ser Glu Asp Ser Ala Val Tyr Tyr Cys
   85 90 95
   Ala Arg Tyr Tyr Asp Asp His Tyr Cys Leu Asp Tyr Trp Gly Gln Gly
   100 105 110
   Thr Thr Leu Thr Val Ser Ser Ala Lys Thr Thr Ala Pro Ser Val Tyr
   115 120 125
   Pro Leu Ala Pro Val Cys Gly Asp Thr Thr Gly Ser Ser Val Thr Leu
   130 135 140
   Gly Cys Leu Val Lys Gly Tyr Phe Pro Glu Pro Val Thr Leu Thr Trp
   145 150 155 160
   Asn Ser Gly Ser Leu Ser Ser Gly Val His Thr Phe Pro Ala Val Leu
   165 170 175
   Gln Ser Asp Leu Tyr Thr Leu Ser Ser Ser Val Thr Val Thr Ser Ser
   180 185 190
   Thr Trp Pro Ser Gln Ser Ile Thr Cys Asn Val Ala His Pro Ala Ser
   195 200 205
   Ser Thr Lys Val Asp Lys Lys Ile Glu Pro Arg Gly Pro Thr Ile Lys
   210 215 220
   Pro Cys Pro Pro Cys Lys Cys Pro Ala Pro Asn Leu Leu Gly Gly Pro
   225 230 235 240
   Ser Val Phe Ile Phe Pro Pro Lys Ile Lys Asp Val Leu Met Ile Ser
   245 250 255
   Leu Ser Pro Ile Val Thr Cys Val Val Val Asp Val Ser Glu Asp Asp
   260 265 270
   Pro Asp Val Gln Ile Ser Trp Phe Val Asn Asn Val Glu Val His Thr
   275 280 285
   Ala Gln Thr Gln Thr His Arg Glu Asp Tyr Asn Ser Thr Leu Arg Val
   290 295 300
   Val Ser Ala Leu Pro Ile Gln His Gln Asp Trp Met Ser Gly Lys Glu
   305 310 315 320
   Phe Lys Cys Lys Val Asn Asn Lys Asp Leu Pro Ala Pro Ile Glu Arg
   325 330 335
   Thr Ile Ser Lys Pro Lys Gly Ser Val Arg Ala Pro Gln Val Tyr Val
   340 345 350
   Leu Pro Pro Pro Glu Glu Glu Met Thr Lys Lys Gln Val Thr Leu Thr
   355 360 365
   Cys Met Val Thr Asp Phe Met Pro Glu Asp Ile Tyr Val Glu Trp Thr
   370 375 380
   Asn Asn Gly Lys Thr Glu Leu Asn Tyr Lys Asn Thr Glu Pro Val Leu
   385 390 395 400
   Asp Ser Asp Gly Ser Tyr Phe Met Tyr Ser Lys Leu Arg Val Glu Lys
   405 410 415
   Lys Asn Trp Val Glu Arg Asn Ser Tyr Ser Cys Ser Val Val His Glu
   420 425 430
   Gly Leu His Asn His His Thr Thr Lys Ser Phe Ser Arg Thr Pro Gly
   435 440 445
   Lys
<210> 3
   <211> 15
   <212> PRT
   <213> Mus musculus
<220>
   <221> Epitope #1
   <222> (1)..(15)
   <223> OKT-3 epitope associated to the genotype HLA-DRB1*0401/*1302.
<400> 3
   Ser Gly Thr Lys Leu Glu Ile Asn Arg Ala Asp Thr Ala Pro Thr
   1 5 10 15
<210> 4
   <211> 16
   <212> PRT
   <213> Mus musculus
<220>
   <221> Epitope #1
   <222> (1)..(16)
   <223> OKT-3 epitope associated to the genotype HLA-DRB1*0401/*1302.
<400> 4
   Gly Ser Gly Thr Lys Leu Glu Ile Asn Arg Ala Asp Thr Ala Pro Thr
   1 5 10 15
<210> 5
   <211> 13
   <212> PRT
   <213> Mus musculus
<220>
   <221> Epitope #2
   <222> (1)..(13)
   <223> OKT-3 epitope associated to the genotype HLA-DRB1*0401/*1302.
<400> 5
   Val Lys Trp Lys Ile Asp Gly Ser Glu Arg Gln Asn Gly
   1 5 10
<210> 6
   <211> 14
   <212> PRT
   <213> Mus musculus
<220>
   <221> Epitope #2
   <222> (1)..(14)
   <223> OKT-3 epitope associated to the genotype HLA-DRB1*0401/*1302.
<400> 6
   Asn Val Lys Trp Lys Ile Asp Gly Ser Glu Arg Gln Asn Gly
   1 5 10
<210> 7
   <211> 15
   <212> PRT
   <213> Mus musculus
<220>
   <221> Epitope #2
   <222> (1)..(15)
   <223> OKT-3 epitope associated to the genotype HLA-DRB1*0401/*1302.
<400> 7
   Ile Asn Val Lys Trp Lys Ile Asp Gly Ser Glu Arg Gln Asn Gly
   1 5 10 15
<210> 8
   <211> 16
   <212> PRT
   <213> Mus musculus
<220>
   <221> Epitope #2
   <222> (1)..(16)
   <223> CKT-3 epitope associated to the genotype HLA-DRB1*0401/*1302.
<400> 8
   Ile Asn Val Lys Trp Lys Ile Asp Gly Ser Glu Arg Gln Asn Gly Val
   1 5 10 15
<210> 9
   <211> 17
   <212> PRT
   <213> Mus musculus
<220>
   <221> Epitope #3
   <222> (1)..(17)
   <223> OKT-3 epitope associated to the genotype HLA-DRB1*0401/*1302.
<400> 9
   Trp Pro Ser Gln Ser Ile Thr Cys Asn Val Ala His Pro Ala Ser Ser
   1               5                   10                  15
   Thr
<210> 10
   <211> 15
   <212> PRT
   <213> Mus musculus
<220>
   <221> Epi t ope #4
   <222> (1)..(15)
   <223> OKT3 epitope associated to the genotype HLA-DRB1*0701/*1601
<400> 10
   Lys Asp Ser Thr Tyr Ser Met Ser Ser Thr Leu Thr Leu Thr Lys
   1               5                   10                  15
<210> 11
   <211> 16
   <212> PRT
   <213> Mus musculus
<220>
   <221> Epitope #4
   <222> (1)..(16)
   <223> OKT3 epitope associated to the genotype HLA-DRB1*0701/*1601
<400> 11
   Lys Asp Ser Thr Tyr Ser Met Ser Ser Thr Leu Thr Leu Thr Lys Asp
   1               5                   10                  15
<210> 12
   <211> 17
   <212> PRT
   <213> Mus musculus
<220>
   <221> Epitope #4
   <222> (1)..(17)
   <223> OKT3 epitope associated to the genotype HLA-DRB1*0701/*1601
<400> 12
   Lys Asp Ser Thr Tyr Ser Met Ser Ser Thr Leu Thr Leu Thr Lys Asp
   1 5 10 15
   Glu
<210> 13
   <211> 17
   <212> PRT
   <213> Mus musculus
<220>
   <221> Epitope #4
   <222> (1)..(17)
   <223> OKT3 epitope associated to the genotype HLA-DRB1*0701/*1601
<400> 13
   Ser Lys Asp Ser Thr Tyr Ser Met Ser Ser Thr Leu Thr Leu Thr Lys
   1 5 10 15
   Asp
<210> 14
   <211> 18
   <212> PRT
   <213> Mus musculus
<220>
   <221> Epitope #4
   <222> (1)..(18)
   <223> OKT-3 epitope associated to the genotype HLA-DRB1*0701/*1601
<400> 14
   Ser Lys Asp Ser Thr Tyr Ser Met Ser Ser Thr Leu Thr Leu Thr Lys
   1 5 10 15
   Asp Glu
<210> 15
   <211> 18
   <212> PRT
   <213> Mus musculus
<220>
   <221> Epitope #4
   <222> (1)..(18)
   <223> OKT-3 epitope associated to the genotype HLA-DRB1*0701/*1601
<400> 15
   Asp Ser Lys Asp Ser Thr Tyr Ser Met Ser Ser Thr Leu Thr Leu Thr
   1 5 10 15
   Lys Asp
<210> 16
   <211> 19
   <212> PRT
   <213> Mus musculus
<220>
   <221> Epitope #4
   <222> (1)..(19)
   <223> OKT-3 epitope associated to the genotype HLA-DRB1*0701/*1601
<400> 16
   Asp Ser Lys Asp Ser Thr Tyr Ser Met Ser Ser Thr Leu Thr Leu Thr
   1 5 10 15
   Lys Asp Glu
<210> 17
   <211> 20
   <212> PRT
   <213> Mus musculus
<220>
   <221> Epitope #4
   <222> (1)..(20)
   <223> OKT-3 epitope associated to the genotype HLA-DRB1*0701/*1601
<400> 17
   Asp Gln Asp Ser Lys Asp Ser Thr Tyr Ser Met Ser Ser Thr Leu Thr
   1 5 10 15
   Leu Thr Lys Asp
   20
<210> 18
   <211> 21
   <212> PRT
   <213> Mus musculus
<220>
   <221> Epitope #4
   <222> (1)..(21)
   <223> OKT-3 epitope associated to the genotype HLA-DRB1*0701/*1601
<400> 18
   Asp Gln Asp Ser Lys Asp Ser Thr Tyr Ser Met Ser Ser Thr Leu Thr
   1               5                   10                  15
   Leu Thr Lys Asp Glu
               20
<210> 19
   <211> 22
   <212> PRT
   <213> Mus musculus
<220>
   <221> Epitope #4
   <222> (1)..(22)
   <223> OKT-3 epitope associated to the genotype HLA-DRB1*0701/*1601
<400> 19
   Thr Asp Gln Asp Ser Lys Asp Ser Thr Tyr Ser Met Ser Ser Thr Leu
   1               5                   10                  15
   Thr Leu Thr Lys Asp Glu
               20
<210> 20
   <211> 15
   <212> PRT
   <213> Mus musculus
<220>
   <221> Epitope #1
   <222> (1)..(15)
   <223> OKT-3 epitope associated to the genotype HLA-DRB1*1101/*1202.
<400> 20
   Ser Gly Thr Lys Leu Glu Ile Asn Arg Ala Asp Thr Ala Pro Thr
   1               5                   10                  15
<210> 21
   <211> 16
   <212> PRT
   <213> Mus musculus
<220>
   <221> Epitope #1
   <222> (1)..(16)
   <223> OKT-3 epitope associated to the genotype HLA-DRB1*1101/*1202
<400> 21
   Gly Ser Gly Thr Lys Leu Glu Ile Asn Arg Ala Asp Thr Ala Pro Thr
   1 5 10 15
<210> 22
   <211> 14
   <212> PRT
   <213> Mus musculus
<220>
   <221> Epitope #3
   <222> (1)..(14)
   <223> OKT-3 epitope associated to the genotype HLA-DRB1*1101/*1202.
<400> 22
   Trp Pro Ser Gln Ser Ile Thr Cys Asn Val Ala His Pro Ala
   1 5 10
<210> 23
   <211> 15
   <212> PRT
   <213> Mus musculus
<220>
   <221> Epitope #3
   <222> (1)..(15)
   <223> OKT-3 epitope associated to the genotype HLA-DRB1*1101/*1202.
<400> 23
   Trp Pro Ser Gln Ser Ile Thr Cys Asn Val Ala His Pro Ala Ser
   1 5 10 15
<210> 24
   <211> 17
   <212> PRT
   <213> Mus musculus
<220>
   <221> Epitope #3
   <222> (1)..(17)
   <223> OKT-3 epitope associated to the genotype HLA-DRB1*1101/*1202.
<400> 24
   Trp Pro Ser Gln Ser Ile Thr Cys Asn Val Ala His Pro Ala Ser Ser
   1               5                   10                  15
   Thr
<210> 25
   <211> 18
   <212> PRT
   <213> Mus musculus
<220>
   <221> Epitope #3
   <222> (1)..(18)
   <223> OKT-3 epitope associated to the genotype HLA-DRB1*1101/*1202.
<400> 25
   Trp Pro Ser Gln Ser Ile Thr Cys Asn Val Ala His Pro Ala Ser Ser
   1               5                   10                  15
   Thr Lys
<210> 26
   <211> 17
   <212> PRT
   <213> Mus musculus
<220>
   <221> Epitope #2
   <222> (1)..(17)
   <223> OKT-3 epitope associated to the genotype HLA-DRB1*0301/*0401.
<400> 26
   Ile Asn Val Lys Trp Lys Ile Asp Gly Ser Glu Arg Gln Asn Gly Val
   1               5                   10                  15
   Leu
<210> 27
   <211> 13
   <212> PRT
   <213> Homo Sapiens
<220>
   <221> Epitope #5
   <222> (1)..(13)
   <223> Interferon-beta-1b epi t ope associated to the genotype HLA-DRB1*0101/*0701
<400> 27
   Gln Phe Gln Lys Glu Asp Ala Ala Leu Thr Ile Tyr Glu
   1 5 10
<210> 28
   <211> 16
   <212> PRT
   <213> Homo Sapi ens
<220>
   <221> Epitope #5
   <222> (1)..(16)
   <223> Interferon-beta-1b epitope associated to the genotype HLA-CRB1*0101/*0701
<400> 28
   Gln Leu Gln Gln Phe Gln Lys Glu Asp Ala Ala Leu Thr Ile Tyr Glu
   1 5 10 15
<210> 29
   <211> 17
   <212> PRT
   <213> Homo Sapiens
<220>
   <221> Epitope #5
   <222> (1)..(17)
   <223> Interferon-beta-1b epitopes associated to the genotype HLA-DRB1*0101/*0701
<400> 29
   Lys Gln Leu Gln Gln Phe Gln Lys Glu Asp Ala Ala Leu Thr Ile Tyr
   1 5 10 15
   Glu
<210> 30
   <211> 11
   <212> PRT
   <213> Homo Sapiens
<220>
   <221> Epi t ope #6
   <222> (1)..(11)
   <223> Interferon-beta-1b epitope associated to the genotype
<400> 30
   Asn Val Tyr His Gln Ile Asn His Leu Lys Thr
   1 5 10
<210> 31
   <211> 12
   <212> PRT
   <213> Homo Sapiens
<220>
   <221> Epitope #6
   <222> (1)..(12)
   <223> Interferon-beta-1b epi t ope associated to the genotype HLA-DRB1*1101/*1404
<400> 31
   Asn Val Tyr His Gln Ile Asn His Leu Lys Thr Val
   1 5 10
<210> 32
   <211> 13
   <212> PRT
   <213> Homo Sapiens
<220>
   <221> Epitope #6
   <222> (1)..(13)
   <223> Interferon-beta-1b epi t ope associated to the genotype HLA-DRB1*1101/*1404
<400> 32
   Asn Val Tyr His Gln Ile Asn His Leu Lys Thr Val Leu
   1 5 10
<210> 33
   <211> 14
   <212> PRT
   <213> Homo Sapiens
<220>
   <221> Epitope #6
   <222> (1)..(14)
   <223> Interferon-beta-1b epitope associated to the genotype HLA-DRB1*1101/*1404
<400> 33
   Asn Val Tyr His Gln Ile Asn His Leu Lys Thr Val Leu Glu
   1 5 10
<210> 34
   <211> 15
   <212> PRT
   <213> Homo Sapiens
<220>
   <221> Epitope #6
   <222> (1)..(15)
   <223> Interferon-beta-1b epitope associated to the genotype HLA-DRB1*1101/*1404
<400> 34
   Asn Val Tyr His Gln Ile Asn His Leu Lys Thr Val Leu Glu Glu
   1 5 10 15
<210> 35
   <211> 16
   <212> PRT
   <213> Homo Sapiens
<220>
   <221> Epitope #6
   <222> (1)..(16)
   <223> Interferon-beta-1b epitope associated to the genotype HLA-DRB1*1101/*1404
<400> 35
   Asn Val Tyr His Gln Ile Asn His Leu Lys Thr Val Leu Glu Glu Lys
   1 5 10 15
<210> 36
   <211> 12
   <212> PRT
   <213> Homo Sapi ens
<220>
   <221> Epitope #6
   <222> (1)..(12)
   <223> Interferon-beta-1b epitope associated to the genotype HLA-DRB1*1101/*1404
<400> 36
   Ala Asn Val Tyr His Gln Ile Asn His Leu Lys Thr
   1 5 10
<210> 37
   <211> 13
   <212> PRT
   <213> Homo Sapiens
<220>
   <221> Epitope #6
   <222> (1)..(13)
   <223> Interferon-beta-1b epitope associated to the genotype HLA-DRB1*1101/*1404
<400> 37
   Ala Asn Val Tyr His Gln Ile Asn His Leu Lys Thr Val
   1 5 10
<210> 38
   <211> 14
   <212> PRT
   <213> Homo Sapiens
<220>
   <221> Epitope #6
   <222> (1)..(14)
   <223> Interferon-beta-1b epitope associated to the genotype HLA-DRB1*1101/*1404
<400> 38
   Ala Asn Val Tyr His Gln Ile Asn His Leu Lys Thr Val Leu
   1 5 10
<210> 39
   <211> 15
   <212> PRT
   <213> Homo Sapiens
<220>
   <221> Epi tope #6
   <222> (1)..(15)
   <223> Interferon-beta-1b epi t ope associated to the genotype HLA-DRB1*1101/*1404
<400> 39
   Ala Asn Val Tyr His Gln Ile Asn His Leu Lys Thr Val Leu Glu
   1 5 10 15
<210> 40
   <211> 16
   <212> PRT
   <213> HOmo Sapiens
<220>
   <221> Epitope #6
   <222> (1)..(16)
   <223> Interferon-beta-1b epitope associated to the genotype HLA-DRB1*1101/*1404
<400> 40
   Ala Asn Val Tyr His Gln Ile Asn His Leu Lys Thr Val Leu Glu Glu
   1 5 10 15
<210> 41
   <211> 17
   <212> PRT
   <213> Homo Sapiens
<220>
   <221> Epitope #6
   <222> (1)..(17)
   <223> Interferon-beta-1b epitope associated to the genotype HLA-DRB1*1101/*1404
<400> 41
   Ala Asn Val Tyr His Gln Ile Asn His Leu Lys Thr Val Leu Glu Glu
   1 5 10 15
   Lys
<210> 42
   <211> 14
   <212> PRT
   <213> Homo Sapiens
<220>
   <221> Epitope #6
   <222> (1)..(14)
   <223> Interferon-beta-1b epitope associated to the genotype HLA-DRB1*1101/*1404
<400> 42
   Leu Ala Asn Val Tyr His Gln Ile Asn His Leu Lys Thr Val
   1 5 10
<210> 43
   <211> 15
   <212> PRT
   <213> Homo Sapiens
<220>
   <221> Epitope #6
   <222> (1)..(15)
   <223> Interferon-beta-1b epitope associated to the genotype HLA-DRB1*1101/*1404
<400> 43
   Leu Ala Asn Val Tyr His Gln Ile Asn His Leu Lys Thr Val Leu
   1 5 10 15
<210> 44
   <211> 16
   <212> PRT
   <213> Homo Sapiens
<220>
   <221> Epitope #6
   <222> (1)..(16)
   <223> Interferon-beta-1b epitope associated to the genotype HLA-DRB1*1101/*1404
<400> 44
   Leu Ala Asn Val Tyr His Gln Ile Asn His Leu Lys Thr Val Leu Glu
   1 5 10 15
<210> 45
   <211> 17
   <212> PRT
   <213> Homo Sapiens
<220>
   <221> Epitope #6
   <222> (1)..(17)
   <223> Interferon-beta-1b epitope associated to the genotype HLA-DRB1*1101/*1404
<400> 45
   Leu Ala Asn Val Tyr His Gln Ile Asn His Leu Lys Thr Val Leu Glu
   1 5 10 15
   Glu
<210> 46
   <211> 16
   <212> PRT
   <213> Homo Sapiens
<220>
   <221> Epitope #6
   <222> (1)..(16)
   <223> Interferon-beta-1b epitope associated to the genotype HLA-DRB1*1101/*1404
<400> 46
   Leu Leu Ala Asn Val Tyr His Gln Ile Asn His Leu Lys Thr Val Leu
   1 5 10 15
<210> 47
   <211> 17
   <212> PRT
   <213> Homo Sapi ens
<220>
   <221> Epitope #6
   <222> (1)..(17)
   <223> Interferon-beta-1b epitope associated to the genotype HLA-DRB1*1101/*1404
<400> 47
   Leu Leu Ala Asn Val Tyr His Gln Ile Asn His Leu Lys Thr Val Leu
   1 5 10 15
   Glu
<210> 48
   <211> 18
   <212> PRT
   <213> Homo Sapiens
<220>
   <221> Epitope #6
   <222> (1)..(18)
   <223> Interferon-beta-1b epitope associated to the genotype HLA-DRB1*1101/*1404
<400> 48
   Leu Leu Ala Asn Val Tyr His Gln Ile Asn His Leu Lys Thr Val Leu
   1 5 10 15
   Glu Glu
<210> 49
   <211> 19
   <212> PRT
   <213> Homo Sapiens
<220>
   <221> Epitope #6
   <222> (1)..(19)
   <223> Interferon-beta-1b epi t ope associated t o the genotype HLA-DRB1*1101/*1404
<400> 49
   Leu Leu Ala Asn Val Tyr His Gln Ile Asn His Leu Lys Thr Val Leu
   1 5 10 15
   Glu Glu Lys
<210> 50
   <211> 18
   <212> PRT
   <213> Homo Sapiens
<220>
   <221> Epitope #6
   <222> (1)..(18)
   <223> Interferon-beta-1b epitope associated to the genotype HLA-DRB1*1101/*1404
<400> 50
   Asn Leu Leu Ala Asn Val Tyr His Gln Ile Asn His Leu Lys Thr Val
   1 5 10 15
   Leu Glu
<210> 51
   <211> 19
   <212> PRT
   <213> Homo Sapiens
<220>
   <221> Epitope #6
   <222> (1)..(19)
   <223> Interferon-beta-1b epi t ope associated to the genotype
<400> 51
   Asn Leu Leu Ala Asn Val Tyr His Gln Ile Asn His Leu Lys Thr Val
   1 5 10 15
   Leu Glu Glu
<210> 52
   <211> 13
   <212> PRT
   <213> Homo Sapiens
<220>
   <221> Epitope #7
   <222> (1)..(13)
   <223> Interferon-beta-1b epi t ope associated to the genotype HLA-DRB1*1101/*1404
<400> 52
   Ile Leu Arg Asn Phe Tyr Phe Ile Asn Arg Leu Thr Gly
   1 5 10
<210> 53
   <211> 15
   <212> PRT
   <213> Homo Sapiens
<220>
   <221> Epi tope #7
   <222> (1)..(15)
   <223> Interferon-beta-1b epitope associated to the genotype HLA-DRB1*1101/*1404
<400> 53
   Val Glu Ile Leu Arg Asn Phe Tyr Phe Ile Asn Arg Leu Thr Gly
   1 5 10 15
<210> 54
   <211> 11
   <212> PRT
   <213> Homo Sapiens
<220>
   <221> Epitope #6
   <222> (1)..(11)
   <223> Interferon-beta-1b epi t ope associated to the genotype HLA-DRB1*0801/*0801
<400> 54
   Asn Val Tyr His Gln Ile Asn His Leu Lys Thr
   1 5 10
<210> 55
   <211> 12
   <212> PRT
   <213> Homo Sapiens
<220>
   <221> Epitope #6
   <222> (1)..(12)
   <223> Interferon-beta-1b epitope associated to the genotype HLA-DRB1*0801/*0801
<400> 55
   Asn Val Tyr His Gln Ile Asn His Leu Lys Thr Val
   1 5 10
<210> 56
   <211> 13
   <212> PRT
   <213> Homo Sapiens
<220>
   <221> Epitope #6
   <222> (1)..(13)
   <223> Interferon-beta-1b epitope associated to the genotype HLA-DRB1*0801/*0801
<400> 56
   Asn Val Tyr His Gln Ile Asn His Leu Lys Thr Val Leu
   1 5 10
<210> 57
   <211> 14
   <212> PRT
   <213> Homo Sapiens
<220>
   <221> Epitope #6
   <222> (1)..(14)
   <223> Interferon-beta-1b epi t ope associated to the genotype HLA-DRB1*0801/*0801
<400> 57
   Asn Val Tyr His Gln Ile Asn His Leu Lys Thr Val Leu Glu
   1 5 10
<210> 58
   <211> 15
   <212> PRT
   <213> Homo Sapiens
<220>
   <221> Epitope #6
   <222> (1)..(15)
   <223> Interferon-beta-1b epitope associated to the genotype HLA-DRB1*0801/*0801
<400> 58
   Asn Val Tyr His Gln Ile Asn His Leu Lys Thr Val Leu Glu Glu
   1 5 10 15
<210> 59
   <211> 12
   <212> PRT
   <213> Homo Sapiens
<220>
   <221> Epitope #6
   <222> (1)..(12)
   <223> Interferon-beta-1b epitope associated to the genotype HLA-DRB1*0801/*0801
<400> 59
   Ala Asn Val Tyr His Gln Ile Asn His Leu Lys Thr
   1 5 10
<210> 60
   <211> 13
   <212> PRT
   <213> Homo Sapiens
<220>
   <221> Epitope #6
   <222> (1)..(13)
   <223> Interferon-beta-1b epitope associated to the genotype HLA-DRB1*0801/*0801
<400> 60
   Ala Asn Val Tyr His Gln Ile Asn His Leu Lys Thr Val
   1 5 10
<210> 61
   <211> 14
   <212> PRT
   <213> Homo Sapiens
<220>
   <221> Epitope #6
   <222> (1)..(14)
   <223> Interferon-beta-1b epitope associ at ed to the genotype HLA-DRB1*0801/*0801
<400> 61
   Ala Asn Val Tyr His Gln Ile Asn His Leu Lys Thr Val Leu
   1 5 10
<210> 62
   <211> 15
   <212> PRT
   <213> Homo Sapiens
<220>
   <221> Epitope #6
   <222> (1)..(15)
   <223> Interferon-beta-1b epitope associated to the genotype HLA-DRB1*0801/*0801
<400> 62
   Ala Asn Val Tyr His Gln Ile Asn His Leu Lys Thr Val Leu Glu
   1 5 10 15
<210> 63
   <211> 13
   <212> PRT
   <213> Homo Sapiens
<220>
   <221> Epitope #6
   <222> (1)..(13)
   <223> Interferon-beta-1b epitope associated to the genotype HLA-DRB1*0801/*0801
<400> 63
   Leu Ala Asn Val Tyr His Gln Ile Asn His Leu Lys Thr
   1 5 10
<210> 64
   <211> 14
   <212> PRT
   <213> Homo Sapiens
<220>
   <221> Epitope #6
   <222> (1)..(14)
   <223> Interferon-beta-1b epitope associated to the genotype HLA-DRB1*0801/*0801
<400> 64
   Leu Ala Asn Val Tyr His Gln Ile Asn His Leu Lys Thr Val
   1 5 10
<210> 65
   <211> 15
   <212> PRT
   <213> Homo Sapiens
<220>
   <221> Epitope #6
   <222> (1)..(15)
   <223> Interferon-beta-1b epitope associated to the genotype HLA-DRB1*0801/*0801
<400> 65
   Leu Ala Asn Val Tyr His Gln Ile Asn His Leu Lys Thr Val Leu
   1 5 10 15
<210> 66
   <211> 16
   <212> PRT
   <213> Homo Sapiens
<220>
   <221> Epitope #6
   <222> (1)..(16)
   <223> Interferon-beta-1b epitope associated to the genotype HLA-DRB1*0801/*0801
<400> 66
   Leu Ala Asn Val Tyr His Gln Ile Asn His Leu Lys Thr Val Leu Glu
   1 5 10 15
<210> 67
   <211> 14
   <212> PRT
   <213> Homo Sapi ens
<220>
   <221> Epitope #6
   <222> (1)..(14)
   <223> Interferon-beta-1b epitope associated to the genotype HLA-DRB1*0801/*0801
<400> 67
   Leu Leu Ala Asn Val Tyr His Gln Ile Asn His Leu Lys Thr
   1 5 10
<210> 68
   <211> 17
   <212> PRT
   <213> Homo Sapiens
<220>
   <221> Epitope #6
   <222> (1)..(17)
   <223> Interferon-beta-1b epitope associated to the genotype HLA-DRB1*0801/*0801
<400> 68
   Leu Leu Ala Asn Val Tyr His Gln Ile Asn His Leu Lys Thr Val Leu
   1 5 10 15
   Glu
<210> 69
   <211> 15
   <212> PRT
   <213> Homo Sapiens
<220>
   <221> Epitope #6
   <222> (1)..(15)
   <223> Interferon-beta-1b epitope associated to the genotype HLA-DRB1*0801/*0801
<400> 69
   Asn Leu Leu Ala Asn Val Tyr His Gln Ile Asn His Leu Lys Thr
   1 5 10 15
<210> 70
   <211> 18
   <212> PRT
   <213> Homo Sapiens
<220>
   <221> Epitope #6
   <222> (1)..(18)
   <223> Interferon-beta-1b epitope associated to the genotype HLA-DRB1*0801/*0801
<400> 70
   Asn Leu Leu Ala Asn Val Tyr His Gln Ile Asn His Leu Lys Thr Val
   1 5 10 15
   Leu Glu
<210> 71
   <211> 11
   <212> PRT
   <213> Homo Sapiens
<220>
   <221> Epitope #7
   <222> (1)..(11)
   <223> Interferon-beta-1b epitope associated to the genotype HLA-DRB1*0801/*0801
<400> 71
   Arg Asn Phe Tyr Phe Ile Asn Arg Leu Thr Gly
   1 5 10
<210> 72
   <211> 13
   <212> PRT
   <213> Homo Sapiens
<220>
   <221> Epitope #7
   <222> (1)..(13)
   <223> Interferon-beta-1b epitope associated to the genotype HLA-DRB1*0801/*0801
<400> 72
   Ile Leu Arg Asn Phe Tyr Phe Ile Asn Arg Leu Thr Gly
   1 5 10
<210> 73
   <211> 14
   <212> PRT
   <213> Homo Sapiens
<220>
   <221> Epitope #7
   <222> (1)..(14)
   <223> Interferon-beta-1b epitope associated to the genotype HLA-DRB1*0801/*0801
<400> 73
   Ile Leu Arg Asn Phe Tyr Phe Ile Asn Arg Leu Thr Gly Tyr
   1 5 10
<210> 74
   <211> 14
   <212> PRT
   <213> Homo Sapiens
<220>
   <221> Epitope #7
   <222> (1)..(14)
   <223> Interferon-beta-1b epitope associated to the genotype HLA-DRB1*0801/*0801
<400> 74
   Glu Ile Leu Arg Asn Phe Tyr Phe Ile Asn Arg Leu Thr Gly
   1 5 10
<210> 75
   <211> 15
   <212> PRT
   <213> Homo Sapiens
<220>
   <221> Epitope #7
   <222> (1)..(15)
   <223> Interferon-beta-1b epitope associated to the genotype HLA-DRB1*0801/*0801
<400> 75
   Val Glu Ile Leu Arg Asn Phe Tyr Phe Ile Asn Arg Leu Thr Gly
   1 5 10 15
<210> 76
   <211> 15
   <212> PRT
   <213> Homo Sapiens
<220>
   <221> Epitope #5
   <222> (1)..(15)
   <223> Interferon-beta-1b epitope associated to the genotype HLA-DRB1*0101/*1401
<400> 76
   Leu Gln Gln Phe Gln Lys Glu Asp Ala Ala Leu Thr Ile Tyr Glu
   1 5 10 15
<210> 77
   <211> 16
   <212> PRT
   <213> Homo Sapiens
<220>
   <221> Epitope #5
   <222> (1)..(16)
   <223> Interferon-beta-1b epitope associated to the genotype HLA-DRB1*0101/*1401
<400> 77
   Gln Leu Gln Gln Phe Gln Lys Glu Asp Ala Ala Leu Thr Ile Tyr Glu
   1 5 10 15
<210> 78
   <211> 17
   <212> PRT
   <213> Homo Sapiens
<220>
   <221> Epitope #5
   <222> (1)..(17)
   <223> Interferon-beta-1b epitope associated to the genotype HLA-DRB1*0101/*1401
<400> 78
   Lys Gln Leu Gln Gln Phe Gln Lys Glu Asp Ala Ala Leu Thr Ile Tyr
   1 5 10 15
   Glu
<210> 79
   <211> 18
   <212> PRT
   <213> Homo Sapiens
<220>
   <221> Epitope #5
   <222> (1)..(18)
   <223> Interferon-beta-1b epitope associated to the genotype HLA-DRB1*0101/*1401
<400> 79
   Ile Lys Gln Leu Gln Gln Phe Gln Lys Glu Asp Ala Ala Leu Thr Ile
   1 5 10 15
   Tyr Glu
<210> 80
   <211> 19
   <212> PRT
   <2113> Homo Sapiens
<220>
   <221> Epitope #5
   <222> (1)..(19)
   <223> Interferon-beta-1b epitope associated to the genotype IHLA-DRBI*0101/*1401
<400> 80
   Ile Lys Gln Leu Gln Gln Phe Gln Lys Glu Asp Ala Ala Leu Thr Ile
   1 5 10 15
   Tyr Glu Met
<210> 81
   <211> 19
   <212> PRT
   <213> Homo Sapiens
<220>
   <221> Epitope #5
   <222> (1)..(19)
   <223> Interferon-beta-1b epitope associated to the genotype HLA-DRB1*0101/*1401
<400> 81
   Glu Ile Lys Gln Leu Gln Gln Phe Gln Lys Glu Asp Ala Ala Leu Thr
   1 5 10 15
   Ile Tyr Glu
<210> 82
   <211> 22
   <212> PRT
   <213> Homo Sapiens
<220>
   <221> Epi tope #5
   <222> (1)..(22)
   <223> Interferon-beta-1b epitope associated to the genotype HLA-CRB1*0101/*1401
<400> 82
   Ile Pro Glu Glu Ile Lys Gln Leu Gln Gln Phe Gln Lys Glu Asp Ala
   1 5 10 15
   Ala Leu Thr Ile Tyr Glu
   20
<210> 83
   <211> 13
   <212> PRT
   <213> Homo Sapiens
<220>
   <221> Epitope #7
   <222> (1)..(13)
   <223> Interferon-beta-1b epitope associated to the genotype HLA-DRB1*0101/*1401
<400> 83
   Ile Leu Arg Asn Phe Tyr Phe Ile Asn Arg Leu Thr Gly
   1 5 10
<210> 84
   <211> 15
   <212> PRT
   <213> Homo Sapiens
<220>
   <221> Epitope #7
   <222> (1)..(15)
   <223> Interferon-beta-1b epitope associated to the genotype HLA-DRB1*0101/*1401
<400> 84
   Val Glu Ile Leu Arg Asn Phe Tyr Phe Ile Asn Arg Leu Thr Gly
   1 5 10 15
<210> 85
   <211> 13
   <212> PRT
   <213> Homo Sapiens
<220>
   <221> Epitope #7
   <222> (1)..(13)
   <223> Interferon-beta-1b epitope associated to the genotype HLA-DRB1*1303/*1501
<400> 85
   Ile Leu Arg Asn Phe Tyr Phe Ile Asn Arg Leu Thr Gly
   1 5 10
<210> 86
   <211> 14
   <212> PRT
   <213> Homo Sapiens
<220>
   <221> Epitope #7
   <222> (1)..(14)
   <223> Interferon-beta-1b epitope associated to the genotype HLA-DRB1*1303/*1501
<400> 86
   Glu Ile Leu Arg Asn Phe Tyr Phe Ile Asn Arg Leu Thr Gly
   1 5 10
<210> 87
   <211> 15
   <212> PRT
   <213> Homo Sapiens
<220>
   <221> Epitope #7
   <222> (1)..(15)
   <223> Interferon-beta-1b epitope associated to the genotype HLA-DRB1*1303/*1501
<400> 87
   Val Glu Ile Leu Arg Asn Phe Tyr Phe Ile Asn Arg Leu Thr Gly
   1               5                   10                  15